(19) Europäisches Patentamt
European Patent Office
Office européen des brevets

(11) Publication number: **0 288 515 B1**

(12) **EUROPEAN PATENT SPECIFICATION**

(45) Date of publication of patent specification: **05.05.93**

(51) Int. Cl.[5]: **A01N 33/12**, A45D 7/04, A61K 7/06, C07D 207/12, C07D 211/76, C07D 295/12, C09B 67/18, C11D 1/58

(21) Application number: **87907051.4**

(22) Date of filing: **24.09.87**

(86) International application number: **PCT/US87/02458**

(87) International publication number: **WO 88/02985 (05.05.88 88/10)**

# (54) OUATERNIZED NITROGEN CONTAINING COMPOUNDS.

(30) Priority: **24.10.86 US 922923**
**28.08.87 US 91008**
**28.08.87 US 91010**
**28.08.87 US 91149**

(43) Date of publication of application: **02.11.88 Bulletin 88/44**

(45) Publication of the grant of the patent: **05.05.93 Bulletin 93/18**

(84) Designated Contracting States: **AT BE CH DE FR GB IT LI LU NL SE**

(56) References cited:
**US-A- 2 945 863**
**US-A- 3 145 147**
**US-A- 3 322 675**
**US-A- 3 332 938**
**US-A- 3 401 005**
**US-A- 3 437 647**
**US-A- 3 754 088**
**US-A- 3 914 403**
**US-A- 4 035 478**
**US-A- 4 540 507**
**US-A- 4 590 069**

(73) Proprietor: **ISP INVESTMENTS INC.**
**818 Washington Street**
**Wilmington, Delaware 19801(US)**

(72) Inventor: **LOGIN, Robert, B.**
**137 Page Drive**
**Oakland, NJ 07436(US)**
Inventor: **CHAUDHURI, Ratan, K.**
**74 Reservoir Avenue**
**Butler, NJ 07405(US)**
Inventor: **TRACY, David, J.**
**209 Comly Road**
**Lincoln Park, NJ 07035(US)**
Inventor: **HELIOFF, Michael, W.**
**485 Otisco Drive**
**Westfield, NJ 07090(US)**

(74) Representative: **Ford, Michael Frederick et al**
**MEWBURN ELLIS 2 Cursitor Street**
**London EC4A 1BO (GB)**

**EP 0 288 515 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

ANGEWANDTE CHEMIE INTERNATIONAL EDITION IN ENGLISH, vol. 11, 1972, Academic Press Inc.; I. HAGEDORN et al., pp. 307–309&NUM;

CHEMISTRY OF ORGANIC COMPOUNDS, 1965, 3rd ed., W.B. Saunders Co., Philadelphia & London; NOLLER , pp. 253, 668, 678&NUM;

CHEMICAL ABSTRACTS, vol. 92, 1980, Columbus, OH (US); STEPHANOU et al., no. 92 : 93862v&NUM;

CHEMICAL ABSTRACTS, vol. 88, 1978, Columbus, OH (US); ARTUS et al., no. 88 : 169962t&NUM;

CHEMICAL ABSTRACTS, vol. 82, 1975, Columbus, OH (US); WATANABE et al., no. 82 : 139964d&NUM;

## Description

In one aspect the invention relates to novel quaternized compounds which possess viscosity enhancing and hair and fiber conditioning properties, which can be used in formulations particularly those containing anionic surfactants. In another aspect the invention relates to novel quaternized compounds having bactericidal properties. Still another aspect of the invention relates to the preparation of said quaternized compounds and in still another aspect, the invention relates to the use of said compounds in several fields of application.

BACKGROUND OF THE INVENTION

The selection of components for hair and skin treating formulations presents numerous difficulties involving compatibility. Several hair treatment and shampoo formulations have been developed which aim to provide conditioning action during cleansing so as to leave the hair soft, manageable and lustrous and thus to eliminate a separate application of creme rinses or conditioning treatments. Problems arise from the limited compatibility of anionic detergents with commercial cationic conditioning agents which precipitate out of solution in shampoo formulations.

Shampoo formulations have employed conventional anionic surfactants such as sodium lauryl sulfate, ammonium lauryl sulfate, amine lauryl sulfates and sodium lauryl sulfate ethers which have been found to be incompatible with most cationic conditioning at effective concentration levels.

Additionally, reproducible thickening for formulations containing anionic detergents such as sodium $\alpha$-olefin sulfonates is very difficult to achieve.

Still another problem encountered in hair conditioning shampoos is one of a preservative nature. It has been found that shampoos, containing inadequate preservative, on standing develop strands of pseudomonas aerouginosa which are clearly visible in the liquid and which may cause scalp infection. Consequently, separate biocidal agents are added to the formulation to prevent development of this bacteria and prevent skin infection. These and many other problems are encountered in the formulation of various shampoos, conditioners and cream rinses. Certain of these difficulties are also encountered in skin lotions, healing salves, mouthwashes, etc.

Accordingly it is an object of this invention to minimize or obviate the above problems while providing additional benefits in hair and skin treating formulations.

Another object of the invention is to provide novel quaternized nitrogen containing compounds having unique properties.

Another object is to provide novel quaternized nitrogen containing compounds having excellent hair conditioning and thickening properties when incorporated into a shampoo and having high compatibility with components of hair and skin treating formulations.

Another object is to provide an economical and commercially feasible method for the preparation of said novel quaternized nitrogen containing compounds.

Still another object is to provide processes for the use of said quaternized compounds.

Hagedon et al, Angew. Chem. int Ed. vol 11, page 307, (1972) discloses a range of hydroxyiminomethyl pyridinium halides and discloses that these compounds reactivate acetylcholinesterase which has been inhibited by phosphorylation. One of these pyridinium halides as the formula

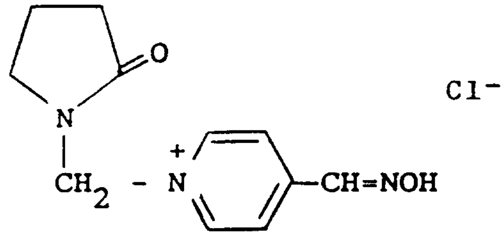

THE INVENTION

In one aspect the present invention provides a quaternized compound having the formula

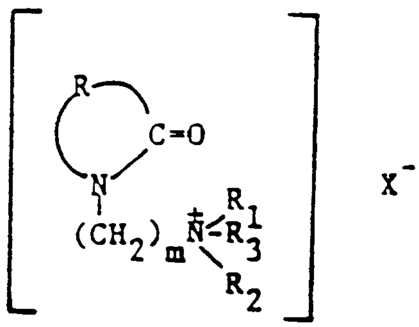

wherein $X^-$ is a chloride, bromide or iodide anion; m is an integer having a value of from 1 to 4; R is $C_3$ to $C_9$ alkylene and is optionally substituted with lower alkyl; and either

(i) $R_1$, $R_2$ and $R_3$ are each independently selected from the group consisting of alkyl, hydroxyalkyl, alkyleneoxyalkyl, alkyleneoxyalkenyl, aryl, alkaryl, aralkyl, aralkenyl, alkyleneamidoalkyl, alkylenecarbamoylalkyl, arylcarbomoylalkyl and arylamidoalkyl radicals each having from 1 to 30 carbon atoms, $R_4-Y-R_7$ or $-(CH_2)_pOR_5$ wherein $R_4$ is alkylene having from 1 to 4 carbon atoms, phenyl or naphthyl optionally substituted with lower alkyl; Y is

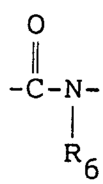

or

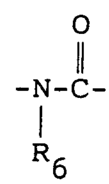

where $R_6$ is hydrogen or lower alkyl; $R_7$ is alkyl of from 1 to 30 carbon atoms, p is an integer having a value from 1 to 4, and $R_5$ is alkyl or alkenyl having from 1 to 30 carbon atoms, and at least one of $R_1$, $R_2$, $R_3$, $R_5$ and $R_7$ having from 8 to 30 carbon atoms, or

(ii) $R_2$ and $R_3$ together with the quaternary nitrogen atom form a 5 to 14 membered heterocyclic ring having from 1 to 2 heteroatoms of the group oxygen, sulfur and nitrogen, while $R_1$ is a said radical having from 1 to 30 carbon atoms or represents a double bond in the quaternized heterocyclic ring formed by $R_2$ and $R_3$, but excluding the compound

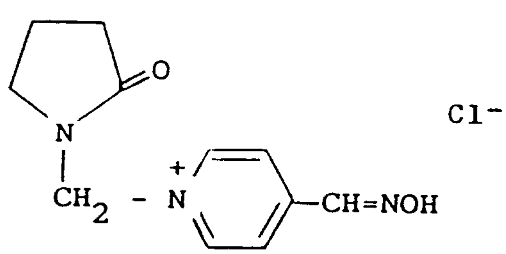

Heterocyclic rings formed by $R_2$ and $R_3$ may be saturated or unsaturated. Examples are the rings of pyrrolidine, morpholine, pyrrole, pyrroline, oxazole, isoxazole, pyrazole, imidazole, pyridine, picoline and diazine. In compounds of this type, $R_1$ may be any of the $R_1$ groups defined above containing from 1 to 30 carbon atoms.

Accordingly compounds included within the scope of this invention include the following subspecies having the specific formulae:

A.

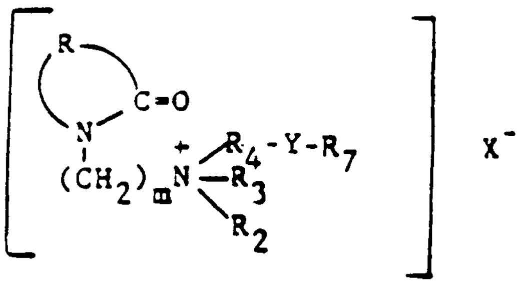

wherein m is an integer having a value of from 1 to 4; R is alkylene having from 3 to 8 carbon atoms and is optionally substituted with $C_1$ to $C_4$ alkyl; $R_4$ is alkylene having from 1 to 4 carbon atoms, phenyl or naphthyl optionally substituted with lower alkyl; Y is

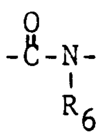

or

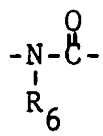

,

where
$R_6$ is hydrogen or lower alkyl; $R_7$ is alkyl of from 1 to 30 carbon atoms; $R_2$ and $R_3$ are each independently $-R_4-Y-R_7$ or a radical selected from alkyl, alkyleneoxyalkyl, alkylhydroxy, aryl, aralkyl, aralkenyl, alkaryl and alkyleneamidoalkyl radicals having from 1 to 30 carbon atoms and at least one of $R_7$, $R_2$ and $R_3$ being a radical having at least 8 carbon atoms and $X^-$ is a chloride, bromide or iodide anion.

Preferred compounds within the above group are those wherein m is 1; R is $-CH_2-CH_2-CH_2-$; at least one of $R_2$ and $R_3$ is methyl;
Y is

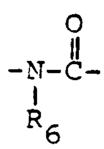

and $X^-$ is a chloride anion. The most preferred compounds of this group are those wherein $R_7$ is alkyl containing at least 8 carbon atoms in a linear chain. Examples of compounds within this preferred group include:

N-methyl-N-allyloxybutyl-N-[(2-pyrrolidonyl)methyl]-N-(3-dodecanamidopropyl) ammonium chloride
N,N-dimethyl-N-[(2-pyrrolidonyl)methyl]-N-(3-octadecanamidopropyl) ammonium chloride
N,N-dimethyl-N-[(2-pyrrolidonyl)methyl]-N-(3-heptadecanamido propyl) ammonium chloride
N-methyl-N-ethyl-N-[(2-pyrrolidonyl)methyl]-N-(3-octadecanamidopropyl) ammonium chloride
N-methyl-N-styryl-N-[(2-pyrrolidonyl)methyl]-N-(3-nonanamido propyl) ammonium chloride
N-methyl-N-tolyl-N-[(2-pyrrolidonyl)methyl]-N-(3-octadecanamidopropyl) ammonium chloride
N-methyl-N-phenyl-N-[(2-pyrrolidonyl)methyl]-N-(3-hexadecanamidopropyl) ammonium chloride
N-decyl-N-methyl-N-[(2-pyrrolidonyl)methyl]-N-(3-dodecanamidopropyl) ammonium chloride
N-methyl-N-[(2-pyrrolidonyl)methyl]-N,N-di(3-hexadecanamidopropyl) ammonium chloride
N,N-dimethyl-N-[(2-pyrrolidonyl)methyl]-N-(undecanamidophenyl) ammonium chloride
N-methyl-N-[(2-pyrrolidonyl)methyl]-N,N-bis(3-undecanamidopropyl) ammonium chloride
N-methyl-N-[(2-pyrrolidonyl)methyl]-N,N-bis(undecanamidophenyl) ammonium chloride
N,N-dimethyl-N-[(2-pyrrolidonyl)methyl]-N-(3-octanamidopropyl) ammonium chloride
N,N-dimethyl-N-[(2-pyrrolidonyl)methyl]-N-(3-dodecanamidopropyl) ammonium chloride
N-butyl-N-methyl-N-[(2-pyrrolidonyl)methyl]-N-(3-dodecanamidopropyl) ammonium chloride
N-phenyl-N-methyl-N-[(2-pyrrolidonyl)methyl]-N-(4-tetradecan amidobutyl) ammonium chloride
N-benzyl-N-methyl-N-[(2-pyrrolidonyl)methyl]-N-(2-hexadecanamidoethyl) ammonium chloride
N-octadecyl-N-methyl-N-[(2-pyrrolidonyl)methyl]-N-(3-octadecanamidopropyl) ammonium

chloride
N,N-dimethyl-N-[(2-pyrrolidonyl)methyl]-N-(3-eicosanamidopropyl) ammonium chloride
N-dodecyl-N-methyl-N-[(2-pyrrolidonyl)methyl]-N-(4-docosanamidobutyl) ammonium chloride
N-methyl-N-[(2-pyrrolidonyl)methyl]-N,N-bis(3-decanamidopropyl) ammonium chloride
N-methyl-N-[(2-pyrrolidonyl)methyl]-N,N-bis(2-dodecanamidoethyl) ammonium chloride
N-methyl-N-[(2-pyrrolidonyl)methyl]-N,N-bis(tetradecanamidomethyl) ammonium chloride
N-methyl-N-[(2-pyrrolidonyl)methyl]-N,N-bis(3-hexadecanamido propyl) ammonium chloride
N-methyl-N-[(2-pyrrolidonyl)methyl]-N,N-bis(3-octadecanamido propyl) ammonium chloride
N-decyl-N-methyl-N-[(2-pyrrolidonyl)methyl]-N-(3-propanamido propyl) ammonium chloride
N-dodecyl-N-methyl-N-[(2-pyrrolidonyl)methyl]-N-(4-propanamidobutyl) ammonium chloride
N-methyl-N-(3-octadecanamidopropyl)-N-[(2-pyrrolidonyl)-methyl]-N-(3-tetradecanamidopropyl) ammonium chloride N-methyl-N-styryl-N-[(2-pyrrolidonyl)methyl]-N-(3-eicosylamidopropyl) ammonium chloride and
N-methyl-N-(hydroxyhexyl)-N-[(2-pyrrolidonyl)methyl]-N-(4-docosanamidobutyl) ammonium chloride, etc.

However, it is to be understood that other halide salts and that the following quaternary compounds represent examples also within the scope of this invention.

N-methyl-N-(hydroxyethyl)-N-[(2-pyrrolidonyl)ethyl]-N-(3-dodecanamidopropyl) ammonium salt
N-ethyl-N-hexadecyl-N-[(2-pyrrolidonyl)methyl]-N-(2-octadecanamidoethyl) ammonium salt
N,N-bis(2-allyloxyethyl)-N-[(2-pyrrolidonyl)methyl]-N-(8-propanamidooctyl) ammonium salt
N-methyl-N-benzyl-N-[(2-pyrrolidonyl)methyl]-N-(14-hexanamidotetradecyl) ammonium salt
N,N-distyryl-N-[(2-pyrrolidonyl)methyl]-N-(3-hexadecanamidopropyl) ammonium salt
N-ethyl-N-[(ethylamino)ethyl]-N-[(2-pyrrolidonyl)methyl]-N-(3-octadecanamidopropyl) ammonium salt
N,N-bis(4-butyloxybutyl)-N-[(2-pyrrolidonyl)methyl]-N(3-ecosanamidopropyl) ammonium salt
N,N-di(3-propanamidopropyl)-N-[(2-pyrrolidonyl)methyl]-N-(4-tetradecanamidobutyl) ammonium salt
N,N-ditolyl-N-[(2-pyrrolidonyl)methyl]-N-(3-octadecanaminopropyl) ammonium salt
N,N-dipropyl-N-[(2-pyrrolidonyl)methyl]-N-(4-decanamidobutyl) ammonium salt
N,N-dioctyl-N-[(2-pyrrolidonyl)methyl]-N-(3-hexanamidopropyl) ammonium salt
N,N-diphenyl-N-[(2-pyrrolidonyl)methyl]-N-(2-decanamidoethyl) ammonium salt
N-[(2-pyrrolidonyl)methyl]-N,N,N-tris(3-tridecanamidopropyl) ammonium salt
N-[2-(2-pyrrolidonyl)ethyl]-N,N,N-tris(pentadecanamidophenyl) ammonium salt
N-methyl-[(2-pyrrolidonyl)methyl]-N,N-bis(tetradecanamidonaphthyl) ammonium salt
N-[(2-decylcarbamoyl)phenyl]-N,N-dimethyl-N-[(2-pyrrolidonyl)methyl] ammonium salt
N-[(2-octadecylcarbamoyl)ethyl]-N,N-dimethyl-N-[(2-pyrrolidonyl)methyl] ammonium salt
N-[(2-pyrrolidonyl)methyl]-N,N,N-tris-(3-octadecanamidopropyl) ammonium salt
N-[(2-pyrrolidonyl)methyl]-N,N,N-tris-(3-tridecanamidopropyl) ammonium salt
N,N-didecyl-N-[(2-pyrrolidonyl)methyl]-N-(6-hexadecanamidohexyl) ammonium salt
N-[(2-pyrrolidonyl)methyl]-N,N,N-tris(3-heptadecanamidopropyl) ammonium salt
N,N-dimethyl-N-[(4-methyl-2-pyrrolidonyl)methyl]-N-(6-pentadecanamidohexyl) ammonium salt
N-methyl-N-[(4-butyl-2-pyrrolidonyl)methyl]-N,N-bis(8-heptadecanamidooctyl) ammonium salt
N,N-dimethyl-N-[(2-piperidinonyl)methyl]-N-(2-heptadecanamidoethyl) ammonium salt
N-methyl-N-[(2-azacycloheptanonyl)methyl]-N,N-bis(octadecanamidobutyl) ammonium salt
N-methyl-N-[(2-azacyclodecanonyl)methyl]-N,N-bis(3-tetradecanamidopropyl) ammonium salt
N-methyl-N-[(2-azacyclononanonyl)methyl]-N,N-bis(dodecanamidohexyl) ammonium salt and others, and other alkylcarbamoylalkyl and N-heterocyclic counterparts of the above alkylamidoalkyl ammonium salts.

B.

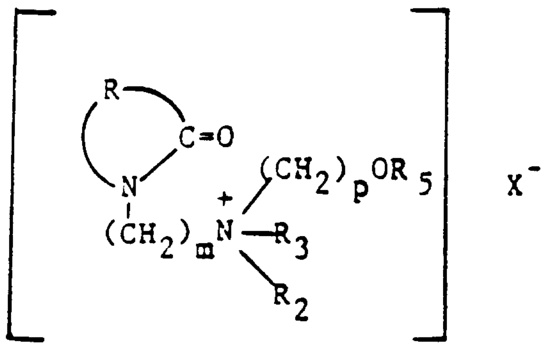

wherein m and p are each integers having a value of from 1 to 4; m is an integer having a value of from 1 to 4; R is alkylene having from 3 to 8 carbon atoms and is optionally substituted with $C_1$ to $C_4$ alkyl; $R_5$ is alkyl or alkenyl having from 1 to 30 carbon atoms; $R_2$ and $R_3$ are each independently $-(CH_2)_pOR_1$, or a radical selected from alkyl, alkylhydroxy, aryl, aralkyl, aralkenyl, alkaryl and alkyl amidoalkyl radicals having from 1 to 30 carbon atoms and at least one of $R_5$, $R_2$ and $R_3$ being a radical having from 8 to 30 carbon atoms; and $X^-$ is a chloride, bromide or iodide anion.

Preferred compounds within group B are those wherein m is 1; R is $-CH_2-CH_2-CH_2-$; at least one of $R_2$ and $R_3$ is lower alkyl and $X^-$ is a chloride anion. The most preferred compounds of this group are those wherein at least one of $R_5$, $R_2$ and $R_3$ contains at least 8 carbon atoms in a linear chain. Examples of the compounds of this invention include:

N,N-dimethyl-N-[(2-pyrrolidonyl)methyl]-N-[3-(octyloxy) propyl] ammonium chloride
N,N-dimethyl-N-[(2-pyrrolidonyl)methyl]-N-[2-(dodecyloxy) ethyl] ammonium chloride
N,N-dibutyl-N-[(2-pyrrolidonyl)methyl]-N-[2-(tetradecyloxy) ethyl] ammonium chloride
N-methyl-N-hydroxyethyl-N-[(2-pyrrolidonyl)methyl]-N-[3-(tetradecyloxy)propyl] ammonium chloride
N-methyl-N-hydroxyethyl-N-[(2-pyrrolidonyl)methyl]-N-[3-(hexadecyloxy)propyl] ammonium chloride
N,N-diphenyl-N-[(2-pyrrolidonyl)methyl]-N-[4-(octadecyloxy) butyl] ammonium chloride
N,N-didodecyl-N-[(2-pyrrolidonyl)methyl)-N-[4-(ethoxy)butyl] ammonium chloride
N-[(2-pyrrolidonyl)methyl]-N,N,N-tris[3-(dodecyloxy)propyl] ammonium chloride
N-[(2-pyrrolidonyl)methyl]-N,N,N-tris[3-(decyloxy)propyl] ammonium chloride
N-butyl-N-benzyl-N-[(2-pyrrolidonyl)methyl]-N-[2(eicosyloxy)ethyl] ammonium chloride
N-methyl-N-tolyl-N-[(2-pyrrolidonyl)methyl]-N-[2-(docosyloxy)ethyl] ammonium chloride
N-phenyl-[(2-pyrrolidonyl)methyl]-N,N-bis[3-(decyloxy) propyl] ammonium chloride
N-ethyl-[(2-pyrrolidonyl)methyl]-N,N-bis[3-(dodecyloxy) propyl] ammonium chloride
N-dodecyl-[(2-pyrrolidonyl)methyl]-N,N-bis[2-(ethyloxy) ethyl] ammonium chloride
N-propyl-[(2-pyrrolidonyl)methyl]-N,N-bis[2-(hexadecyloxy) ethyl] ammonium chloride
N-octadecyl-[(2-pyrrolidonyl)methyl]-N,N-bis[3-(octadecyloxy)propyl] ammonium chloride
N-propyl-N-styryl-[(2-pyrrolidonyl)methyl]-N-[4-(dodecyloxy) butyl] ammonium chloride
N-butyl-N-octadecyl-[(2-pyrrolidonyl)methyl]-N-[2-(allyloxy) ethyl] ammonium chloride
N-propyl-N-ethylamidoethyl-[(2-pyrrolidonyl)methyl]-N-[4-(hexenyloxy)butyl] ammonium chloride
N,N-dimethyl-[(2-pyrrolidonyl)methyl]-N-[4-(dodecenyloxy) butyl] ammonium chloride
N,N-dimethyl-[(2-pyrrolidonyl)methyl]-N-[3-(tetradecenyloxy) propyl] ammonium chloride, etc.

However, it is to be understood that other halide salts of the following quaternary compounds represent examples also within the scope of this invention.

N-methyl-N-ethyl-[2-(2-pyrrolidonyl)ethyl]-N-[3-(dodecyloxy) propyl] ammonium salt
N-ethyl-N-hexadecyl-[4-(2-pyrrolidonyl)butyl]-N-[3-(octadecyloxy)propyl] ammonium salt
N,N-dimethyl-[2-(2-pyrrolidonyl)ethyl]-N-[3-(octyloxy) propyl] ammonium salt
N,N-dimethyl-[3-(2-pyrrolidonyl)propyl]-N-[4-(dodecenyloxy) butyl] ammonium salt
N-methyl-N-dodecyl-[4-(2-piperidonyl)butyl]-N-[3-(propyloxy) propyl] ammonium salt
N-hydroxyethyl-N-ethyl-[(2-piperidonyl)methyl]-N-[2-(decyloxy)ethyl] ammonium salt
N-benzyl-N-butyl-[4-(2-azacycloheptanonyl)butyl]-N-[3-(dodecyloxy)propyl] ammonium salt
N-benzyl-N-methyl-[(2-piperidinonyl)methyl]-N-[2-(octenyloxy)ethyl] ammonium salt
N-styryl-N-methyl-[(2-azacyclononanonyl)methyl]-N-[4-(hexadecyloxy)butyl] ammonium salt
N-phenyl-[(2-azacyclodecanonyl)methyl]-N,N-bis[3-(dodecyloxy)propyl] ammonium salt

N-hexylamidoethyl-N-methyl-[3-(2-piperidonyl)propyl](3-octadecyloxy)propyl] ammonium salt
N,N-dimethyl-N-[(2-azacyclononanoyl)methyl]-N-[3-(triacontyloxy)propyl] ammonium salt
N,N-didodecyl-N-[3-(2-azacycloheptanonyl)propyl]-N-[2-(ethyl oxy)ethyl] ammonium salt
N,N-dibutyl-N-[4-(2-pyrrolidonyl)butyl]-N-[4-(tetradecenyloxy)butyl] ammonium salt
N-[2-(2-pyrrolidonyl)ethyl]-N,N,N-tris[2-(octadecyloxy) ethyl] ammonium salt
N-[4-(2-piperidonyl)butyl]-N,N,N-tris[2-(decenyloxy)ethyl] ammonium salt
N-[(2-azacycloheptanonyl)methyl]]-N,N,N-tris[4-(tetradecyloxy)butyl] ammonium salt
N,N-dibutylhydroxy-N-[(2-azacycloheptanonyl)methyl]-N-[3-(nonyloxy)propyl] ammonium salt
N,N-didecyl-N-[(2-azacyclononanoyl)methyl]-N-[3-(hexadecyloxy)propyl] ammonium salt
N-[(4-ethyl-2-azacyclononanoyl)methyl]-N,N,N-tris[4-(octadecyloxy)butyl] ammonium salt
N,N-dimethylamidoethyl-[(3,4-dimethyl-2-pyrrolidonyl)methyl] -N-[3-(octadecyloxy)propyl] ammonium salt
N-xylyl-N-[(3,4,5-tributyl-2-pyrrolidonyl)methyl]-N,N-bis-[3-(hexadecyloxy)propyl] ammonium salt
N-N-dimethyl-N-[(4-methyl-2-piperidinonyl)ethyl]-N-[2-(hexadecyloxy)ethyl] ammonium salt
N-p-ethylphenyl-N-[(3,4,5-trimethyl-2-piperidonyl)methyl]-N, N-bis-[3-(propyloxy)propyl] ammonium salt
N,N-tetradecyl-N-[(4-butyl-2-piperidonyl)methyl]-N-[3-(octyl oxy)propyl] ammonium salt
N-(8-octamidooctyl)-N-[(4,5-diethyl-2-azacyclononanonyl) methyl]-N,N-bis-[3-(dodecyloxy)-propyl] ammonium salt and others.

C.

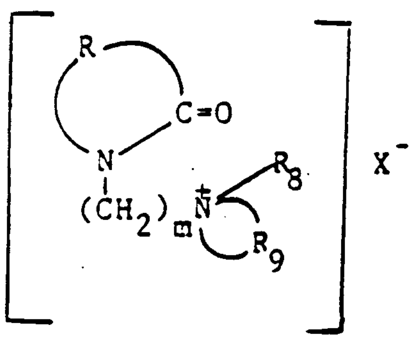

wherein $X^-$ is a chloride, bromide or iodide anion; m is an integer having a value of from 1 to 4; R is alkylene having from 3 to 8 carbon atoms and is optionally substituted with $C_1$ to $C_4$ alkyl; $R_8$ forms a double bond in the heterocyclic ring with the quaternized nitrogen or is alkyl, alkyleneoxyalkyl, alkylhydroxy, aryl, aralkyl, aralkenyl, alkaryl and alkylamidoalkyl, said groups containing up to 30 carbon atoms; and $R_9$ together with the quaternary nitrogen atom forms a 5 to 10 membered monocyclic ring, a 9 to 10 membered bicyclic ring or a 13 to 14 membered tricyclic ring, at least one of said rings containing from 1 to 2 hetro atoms selected from the group of nitrogen, sulfur and oxygen and being saturated or unsaturated as may occur for example in the rings of thiazirine, N-methyl-thiazine, N-methyl-oxazine, thiazepine, N-ethyl-oxazocine, diazocine, N-methylazonine, antipyrine, conyrine, coniine, collidine, diazine, imidazole, isoxazole, lutidine, N-methyl hexahydropyridine, morpholine, oxazole, picoline, piperidine, pyrine, pyrazole, pyridine, pyrrolidine, pyrrole, pyrroline, N-ethylbenzopyrrole, N,N'-dimethyl-dipyrrylmethane, decahydroquinoline, methyl indole, nicotine, quinoline, quinaldine, acridine and carbazole.

As indicated, a heterocyclic ring of the $R_9$ moiety may contain carbonyl substitution or the ring itself can be substituted with a lower alkyl, amino or amido group.

Preferred compounds within group C are those wherein m is 1; R is $-CH_2-CH_2-CH_2-$; $R_8$ is a double bond or a group having $C_8$ to $C_{22}$ carbon atoms, most preferably alkyl and $X^-$ is a chloride anion. The most preferred compounds of this group are those therein $R_9$ together with the quaternized nitrogen forms a oxazolidinyl, pyrrolidinyl, piperidinyl, morpholinyl, pyridinyl or imidazolinyl ring. Examples of the present polycyclic compounds include:

N-hexadecyl-N-[(2-pyrrolidonyl)methyl] pyrrolium chloride N-octadecyl-N-[(2-pyrrolidonyl)methyl] pyrrolidinium chloride
2-ethyl-N-[(2-piperidonyl)methyl] 2-oxazolinium chloride
N-octadecyl-N-[(2-pyrrolidonyl)methyl] oxazolidinium chloride
5-methyl-N-[(2-pyrrolidonyl)methyl] isooxazolium chloride

N – ethyl – N – [(2 – piperidonyl)methyl] isooxazolidinium chloride
2 – methyl – N – [(2 – pyrrolidonyl)methyl] thiazolinium chloride
N – dodecyl – N – [(2 – pyrrolidonyl)methyl] thiazolidinium chloride
1 – (2 – hydroxyethyl) – 2 – heptadecyl – 1(or 3) – [(2 – pyrrolidonyl) methyl] – 2 – imidazolinium chloride
1 – (2 – hydroxyethyl) – 2 – pentadecyl – 1(or 3) – [(2 – pyrrolidonyl) methyl] – 2 – imidazolinium chloride
1 – (2 – hydroxyethyl) – 2 – heneicosyl – 1(or 3) – [(2 – pyrrolidonyl) methyl] – 2 – imidazolinium chloride
1 – (2 – hydroxyethyl) – 2 – tridecyl – 1(or 3) – [(2 – pyrrolidonyl) methyl] – 2 – imidazolinium chloride
1 – methyl – 2 – dodecyl – 1(or 3) – [(2 – pyrrolidonyl)methyl] – 2 – imidazolinium chloride
4 – hexadecyl – N – [(2 – pyrrolidonyl)methyl] pyridinium chloride
N – octadecyl – N – [(2 – pyrrolidonyl)methyl] piperidinium chloride
1,4 – diethyl – 1(or 4) – [(2 – pyrrolidonyl)methyl] piperazinium chloride
2 – methyl – 1(or 3) – [(2 – pyrrolidonyl)methyl] pyrimidinium chloride
N – octadecyl – N – [(2 – pyrrolidonyl)methyl] morpholinium chloride
N – octadecyl – N – [(2 – azacycloheptanonoyl)methyl] morpholinium chloride
4 – ethyl – N – [(2 – pyrrolidonyl)methyl] quinolinium chloride
4 – methyl – N – [(2 – pyrrolidonyl)methyl] tetrahydroquinolinium chloride
4 – ethyl – N – [(2 – pyrrolidonyl)methyl] isoquinolinium chloride
4 – ethyl – N – [(2 – pyrrolidonyl)methyl] tetrahydroisoquinolinium chloride
N – methyl – N – [(2 – pyrrolidonyl)methyl] benzisooxazolinium chloride
N – ethyl – N – [(2 – pyrrolidonyl)methyl] benzthiazolinium chloride
N – octadecyl – N – [(2 – pyrrolidonyl)methyl] indolinium chloride
N – dodecyl – N – [(2 – pyrrolidonyl)methyl] indolinium chloride
2 – hexyl – N – [(2 – pyrrolidonyl)methyl] benzimidazolinium chloride
N – ethyl – N – [(2 – pyrrolidonyl)methyl] acridinium chloride
N – methyl – N – [(2 – pyrrolidonyl)methyl] – 9 – acridinium chloride
N – octadecyl – N – [(2 – pyrrolidonyl)methyl] carbazolinium chloride
N – [(2 – pyrrolidonyl)methyl] thiazepinium chloride
N – methyl – N – [(2 – pyrrolidonyl)methyl] thiazinium chloride
N – [(2 – pyrrolidonyl)methyl] antipyrinium chloride
N – [(2 – pyrrolidonyl)methyl] diazinium chloride
N – propyl – N – [4 – (2 – pyrrolidonyl)butyl] pyrazolinium chloride
N – dodecyl – N – [2 – (2 – pyrrolidonyl)ethyl] pyrrolinium chloride
1,2 – bis – [2 – (2 – pyrrolidonyl)ethyl] antipyrinium chloride
N,N' – bis – [(2 – pyrrolidonyl)methyl] nicotinium chloride
methylene – bis – N – methyl – N – [(2 – pyrrolidonyl)methyl] pyrrolium chloride
N,N' – dimethyl – N,N' – bis – [(2 – pyrrolidonyl)methyl] diazacyclohendacanium chloride
N,N' – diethyl – N,N' – bis – [(2 – pyrrolidonyl)methyl] diazacyclotetradecanium chloride
and other lower alkyl substituted species of quaternized halides of N – haloalkyl 2 – pyrrolidones, 2 – piperidones, 2 – azacycloheptanones, 2 – azacyclooctanones, 2 – azacyclononanones, and 2 – azacyclododecanones

More specifically, the products of this group can be described by the formula:

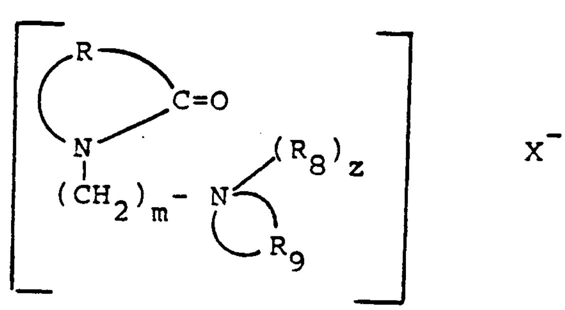

wherein z has a value of zero when the quaternized nitrogen atom is bonded in the heterocyclic ring by a double bond and a single bond and has a value of one when the quaternized nitrogen atom is bonded in the heterocyclic ring by two single bonds. All of the remaining definitions of R, $R_9$, $R_8$ and m being the same as set forth above.

D.

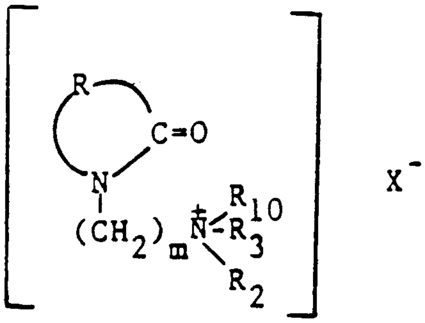

wherein m is an integer having a value of from 1 to 4; R is alkylene having from 3 to 8 carbon atoms and is optionally substituted with $C_1$ to $C_4$ alkyl; $R_{10}$, $R_2$ and $R_3$ are each independently selected from the group of alkyl, alkylhydroxy, aryl, aralkyl, alkaryl and aralkenyl, said groups each having from 1 to 30 carbon atoms and at least one of $R_{10}$, $R_2$ and $R_3$ is a radical having from 8 to 30 carbon atoms; and $X^-$ is a chloride, bromide or iodide anion.

Preferred compounds within group D are those wherein m is 1; R is $-CH_2-CH_2-CH_2-$; at least one of $R_{10}$, $R_2$ and $R_3$ is methyl and $X^-$ is a chloride anion. The most preferred compounds of this group are those wherein at least one of $R_2$ and $R_3$ is alkyl containing from 8 to 22 carbon atoms. Examples of compounds within this preferred group include:

N,N-dimethyl-N-nonyl-[(2-pyrrolidonyl)methyl] ammonium chloride
N,N-dimethyl-N-decyl-[(2-pyrrolidonyl)methyl] ammonium chloride
N,N-dimethyl-N-dodecyl-[(2-pyrrolidonyl)methyl] ammonium chloride
N,N-dimethyl-N-tetradecyl-[(2-pyrrolidonyl)methyl] ammonium chloride
N,N-dimethyl-N-hexadecyl-[(2-pyrrolidonyl)methyl] ammonium chloride
N,N-dimethyl-N-octadecyl-[(2-pyrrolidonyl)methyl] ammonium chloride
N,N-dimethyl-N-eicosyl-[(2-pyrrolidonyl)methyl] ammonium chloride
N,N-dimethyl-N-docosyl-[(2-pyrrolidonyl)methyl] ammonium chloride
N-methyl-N,N-didecyl-[(2-pyrrolidonyl)methyl] ammonium chloride
N-methyl-N,N-didodecyl-[(2-pyrrolidonyl)methyl] ammonium chloride
N-methyl-N,N-ditetradecyl-[(2-pyrrolidonyl)methyl] ammonium chloride
N-methyl-N,N-dihexadecyl-[(2-pyrrolidonyl)methyl] ammonium chloride
N-methyl-N,N-dioctadecyl-[(2-pyrrolidonyl)methyl] ammonium chloride
N-methyl-N,N-dieicosyl-[(2-pyrrolidonyl)methyl] ammonium chloride and
N-methyl-N,N-didocosyl-[(2-pyrrolidonyl)methyl] ammonium chloride

However, it is to be understood that other halide salts and that the following quaternary compounds represent examples also within the scope of this invention.

N-methyl-N-ethyl-N-dodecyl-N-[(2-pyrrolidonyl)ethyl] ammonium salt
N-ethyl-N-styryl-N-octadecyl-[(2-pyrrolidonyl)methyl] ammonium salt
N,N-dimethyl-N-xylyl-[(2-pyrrolidonyl)methyl] ammonium salt
N,N-diphenyl-N-octadecyl-[(2-pyrrolidonyl)propyl] ammonium salt
N-methyl-N,N-bis(dodecylhydroxy)-[(2-pyrrolidonyl)ethyl] ammonium salt
N-ethylhydroxy-N-ethyl-N-pyrrolidinyl-[(2-pyrrolidonyl)-butyl] ammonium salt
N,N-dibutyl-N-hexadecenyl-[(2-pyrrolidonyl)methyl] ammonium salt
N-methyl-N,N-bis(3-butenylphenyl)-[(2-pyrrolidonyl)ethyl] ammonium salt
N-methyl-N,N-bis(decyl)-[(2-pyrrolidonyl)propyl] ammonium salt
N-dimethyl-N,N-ditolyl-[(2-pyrrolidonyl)butyl] ammonium salt
N,N-dimethyl-N-triacontyl-[(2-pyrrolidonyl)methyl] ammonium salt
N,N-dimethyl-N-octadecylphenyl-[(2-pyrrolidonyl)methyl] ammonium salt
N,N-didecyl-N-hexadecyl-[(2-pyrrolidonyl)methyl] ammonium salt
N,N,N-tris(octadecyl)-[(2-pyrrolidonyl)ethyl] ammonium salt
N,N-diethyl-N-octadecyl—[(4-methyl-2-pyrrolidonyl)methyl] ammonium salt
N,N,N-tris(3,5-diethylphenyl)-[(2-pyrrolidonyl)methyl] ammonium salt
N-methyl-N,N-dihexadecyl-[(4-butyl-2-pyrrolidonyl)methyl] ammonium salt
N,N-dimethyl-N-hexadecyl-[(2-piperidinonyl)propyl] ammonium salt
N-methyl-N,N-dioctadecyl-[(2-azacycloheptanoyl)methyl] ammonium salt
N-methyl-N,N-ditetradecyl-[(2-azacyclononanoyl)methyl] ammonium salt

N-methyl-N,N-didodecyl-[(2-azacycldecanonyl)ethyl] ammonium salt and others.

The present quaternary compounds possess unique properties, among which is their ability to build viscosity, e.g. for liquids having a viscosity less than 50 centipoises, and also to provide a hair and skin conditioning capability in cosmetic formulations containing anionic surfactants. In the context of this application, "conditioning" includes the functions of moisturizing, softening, cleansing, penetrating, disinfecting, luster enhancing, hair compatibility, dye leveling, thickening, dye retention and others. These compounds are highly compatible with $\alpha$-olefin sulfonates and anionic surfactant salts conventionally employed in shampoos, skin lotions and textile treating products. Their compatibility is such that up to 5% by weight or more of the quaternized compounds can be incorporated in the formulation, a characteristic which permits the formation of effective formulations as liquids or gels. In contrast, most prior quaternary viscosity building conditioning compounds are incorporable only up to 0.5 or 1 wt. percent based on total anionic formulations. The pyrrolidonyl compounds which contain an octadecyl moiety on the quaternary nitrogen are particularly outstanding for their compatability, viscosity building and fiber and hair conditioning properties. The pyrrolidonyl products having a hexadecyl or dodecyl moiety or, those which contain sulfur in the group attached to, the quaternized nitrogen possess antibacterial and fungicidal properties and can be used in the control of dandruff or as a highly compatible preservative in shampoo, hair conditioners and hand or body lotions. It is contemplated that mixtures of pyrrolidonyl compounds, such as those containing $C_{16}$ and $C_{18}$ alkyl terminally substituted compounds, can be employed in shampoos, hair conditioners, skin lotions and textile softeners as an agent which incorporates thickening, scroop, conditioning and preservative qualities in one additive; thus eliminating the need for separate chemical components to accomplish these individual needs. These mixtures may also be used to control dandruff or bacterial infections of the scalp and body skin. Generally, the quaternary compounds of this invention are mixed with a standard formulation of shampoo, cream rinse, hand or body lotion or creams, mouthwash, etc., in an effective amount which ranges from between 0.05 to 8% by weight, preferably between 0.5 and 5% by weight, of the total formulation. The compatability of the present compounds with anionic $\alpha$-olefin sulfonates is surprising since most anionic compounds cause precipitation of cationic agents. However, the present compounds in concentrations up to 5% by weight show no tendency to precipitate after extended periods including periods up to 6 months or more.

The quaternary lactam products of this invention are prepared by an economically feasible process which involves the reaction between a N-haloalkyl lactam having a 5 to 10 membered ring and a tertiary amine coreactant. A general equation for the preparation is defined by the equation:

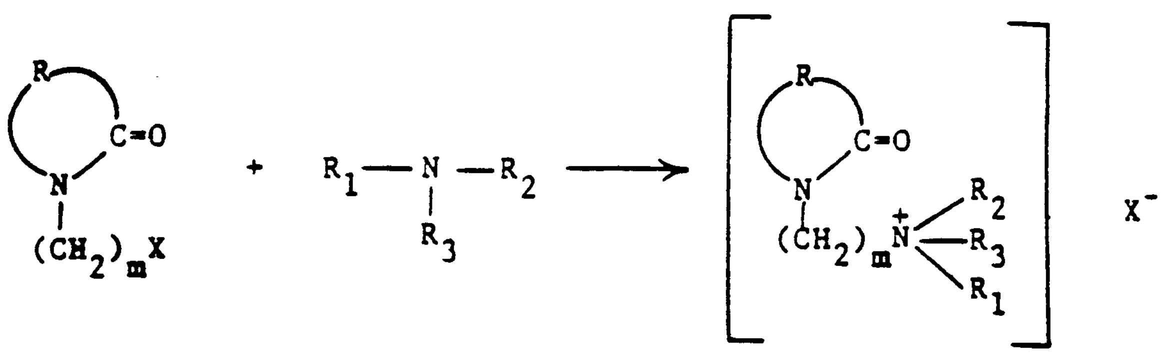

wherein m, R, $R_1$, $R_2$, $R_3$ and $X^-$ are as defined above and X is chloro, bromo or iodo.

Examples of suitable N-haloalkyl lactam reactants include the N-chloroethyl, N-chloropropyl, N-chlorobutyl, N-chloromethyl, N-bromomethyl and N-iodopropyl derivatives of 2-pyrrolidone, 4-methyl-2-pyrrolidone, 4-butyl-2-pyrrolidone, 2-piperidone, methyl-2-piperidone, 2-azacycloheptanone, 2-azacyclooctanone, 2-azacyclononanone, 2-azacyclodecanone and $C_1$ to $C_4$ alkyl substituted derivatives located on an alkylene group in the heterocyclic ring of these lactams. Mixtures of these lactam reactants can also be employed to provide a correspondingly mixed quaternary product, if desired. Of these lactam reactants the N-halomethyl-2-pyrrolidones, N-halomethyl 2-piperidones and 2-azacycloheptanones are preferred and the N-chloromethyl lactams are most preferred.

More specifically, synthesis for the above subspecies can be defined as follows.

For subspecies A:

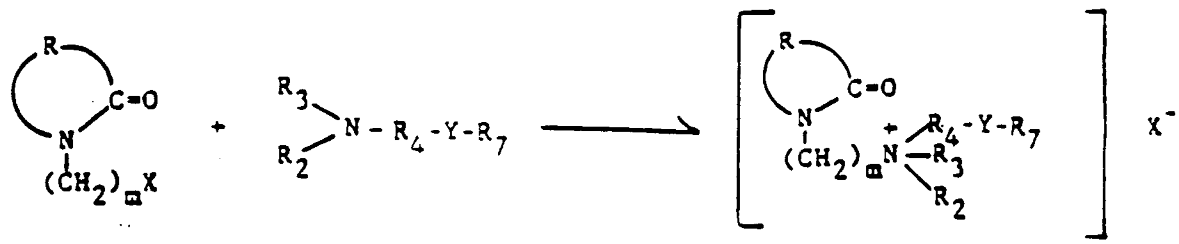

wherein m, R, $R_7$, $R_4$, $R_2$, $R_3$, Y and $X^-$ are as defined above and X is chloro, bromo or iodo.

Preferred tertiary amido amine coreactants employed in the present process include methyl and ethyl tertiary amido amines having one or more $C_8$ to $C_{22}$ alkyl groups, for example
N-methyl-N-dodecyl-N-(3-decanamidopropyl) amine;
N-ethyl-N-methyl-N-(3-dodecanamidopropyl) amine;
N,N-dimethyl-N-(3-tetradecanamidobutyl) amine;
N,N-bis(hydroxyethyl)-N-(2-octanamidoethyl) amine,
N-ethyl-N-decyl-N-(3-octadecanamidopropyl) amine;
N-butyl-N-methyl(2-hexadecanamidoethyl) amine;
N,N-dimethyl-N-(4-decanamidobutyl) amine;
N-methyl-N-tetradecyl-N-(butyramidobutyl) amine;
N-methyl-N,N-(3-tetradecanamidopropyl) amine;
N-methyl-N-phenyl-N-(3-hexadecanamidopropyl) amine;
N,N-dibenzyl-N-(2-octadecanamidoethyl) amine;
N-methyl-N,N-bis(3-undecanamidopropyl) amine
N-propyl-N,N-bis(3-undecanamidophenyl) amine etc. Other suitable coreactants including any of the carbamoyl amine counterparts of the above compounds. e.g. N-butyl-N,N-bis[(decylcarbamoyl)phenyl] amine, and any of the tertiary amines corresponding to the aforenamed quaternized products of this invention, are also suitably employed.

Also included in the scope of this invention are tertiary amido amines or carbamoyl amines wherein a $C_8$ to $C_{30}$ alkyl group represented by $R_2$, $R_3$ or $R_7$ is itself substituted with alkyl or alkoxy of 1 to 4 carbon atoms, phenyl, tolyl, xylyl, halo substituted mono cyclic aromatics, styryl, benzyl, amido. Tris $C_8$ to $C_{22}$ alkylamidoalkyl coreactants can also be employed in the reaction, examples of which include tri(3-hexadecanamidopropyl) amine, tri(4-octadecanamidobutyl) amine, tri(2-dodecanamidoethyl) amine, tri(3-decanamidopropyl) amine, etc.

The most preferred amino coreactants of this invention are those wherein $R_3$ is methyl, $R_2$ is methyl or $R_4-Y-R_7$ and at least one of $R_2$, $R_3$ and $R_7$ is $C_8$ to $C_{22}$ alkyl.

Synthesis for subspecies B can be described as follows:

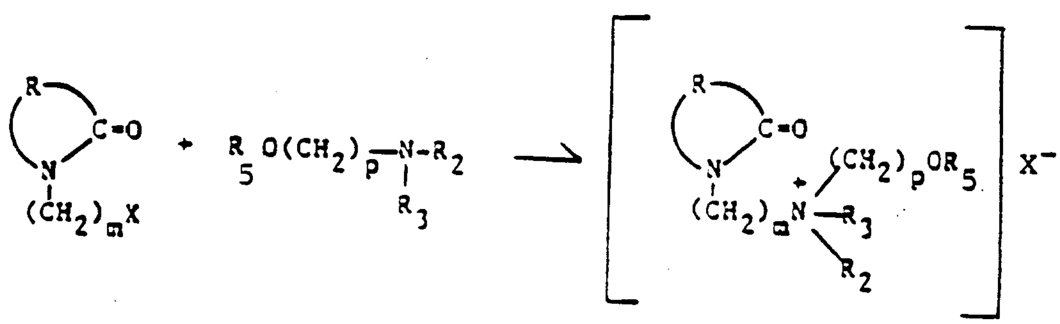

wherein m, p, R, $R_5$, $R_2$, $R_3$ and $X^-$ are as defined above and X is chloro, bromo or iodo.

The tertiary ether amines employed in the above equation include reactive methyl and ethyl tertiary species containing at least one group having 8 to 22 carbon atoms, for example N-methyl-N,N-didecyloxy propyl amine, N,N-dimethyl-N-octadecyloxy propyl amine, N,N-dimethyl-N-dodecyloxy ethyl amine, N,N-dimethyl-N-hexadecyloxy butyl amine, N,N-dimethyl-N-eicosyloxy propyl amine,

N-ethyl-N,N-dihexadecyloxy propyl amine and others including any of the ether amines corresponding to the forenamed quaternized products of this invention, e.g. N-methyl-N-ethoxy-N-hexadecyloxy propyl amine, N-phenyl-N,N-didecyloxy propyl amine, N-dodecyl-N,N-diethoxy ethyl amine, N-octadecylamidopropyl-N,N-dimethoxy ethyl amine, etc. However, also included in the scope of this invention are reactive ether amines wherein a $C_8$ to $C_{30}$ group is itself substituted with alkoxy of 1 to 4 carbon atoms, phenyl, benzyl, amido, as well as tertiary ether amines having 1 to 2 aryl groups, eg. phenyl, benzyl, tolyl, xylyl, etc., such as a dibisphenyl ether amine, a ditolyl ether amine, a benzyl phenyl ether amine, and halo substituted phenyl ether amines. Tri- $C_8$ to $C_{22}$ alkoxyalkyl amines can also be employed in the reaction, examples of which include tris(hexadecyloxypropyl) amine, tris(octadecyloxybutyl) amine, tris(dodecyloxyethyl) amine, tris(decyloxypropyl) amine, etc.

The preferred ether amine reactants of this invention are those defined by the formula:

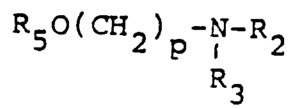

wherein $R_2$ is methyl, $R_3$ is methyl or $-(CH_2)_pOR_1$ and $R_5$ is $C_8$ to $C_{22}$ alkyl.

Synthesis for subspecies C is described as follows:

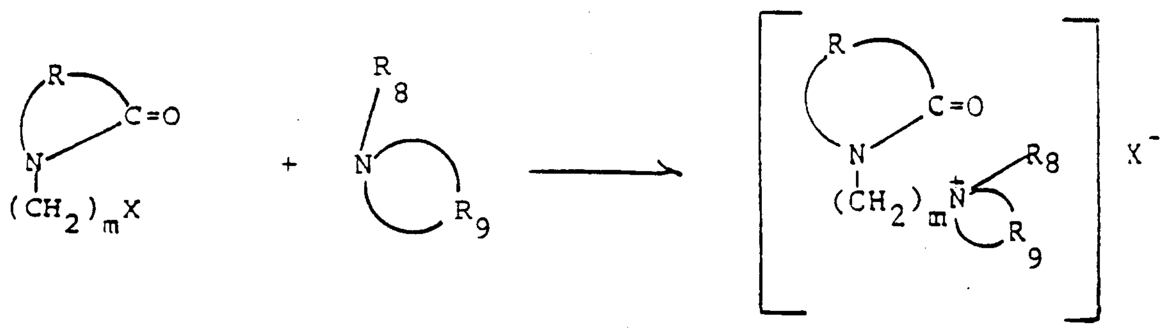

wherein m, R, $R_8$, $R_9$ and $X^-$ are as defined above and X is chloro, bromo or iodo.

Examples of suitable lactam reactants include the N-chloromethyl, N-bromomethyl and N-iodomethyl derivatives of 2-pyrrolidone, 4-methyl-2-pyrrolidone, 4-butyl-2-pyrrolidone, 2-piperidone, methyl-2-piperidone, 2-azacycloheptanone, 2-azacyclooctanone, 2-azacyclononanone, 2-azacyclodecanone and $C_1$ to $C_4$ alkyl derivatives substituted on an alkylene group in the heterocyclic ring of these lactams. Mixtures of these lactam reactants can also be employed to provide a correspondingly mixed quaternary product, if desired. Of these lactam reactants the N-halomethyl-2-pyrrolidones and N-halomethyl caprolactams are preferred and the N-chloromethyl lactams are most preferred.

Suitable nitrogen heterocyclic amino coreactants include any of the compounds defined by the aforementioned

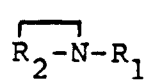

heterocyclic group. Preferred of this group are pyridine, oxazolidine and N-alkyl substituted pyrrolidines, piperidines, morpholines and imidazoles wherein most preferably the alkyl bonded to the heterocyclic nitrogen contains from 8 to 20 carbon atoms. Also, the most preferred heterocyclic amine coreactants of this invention are those wherein $R_2$ is a hydrocarbon group having a total of 4 or 5 carbon atoms.

The preparation for subspecies D can be described as follows:

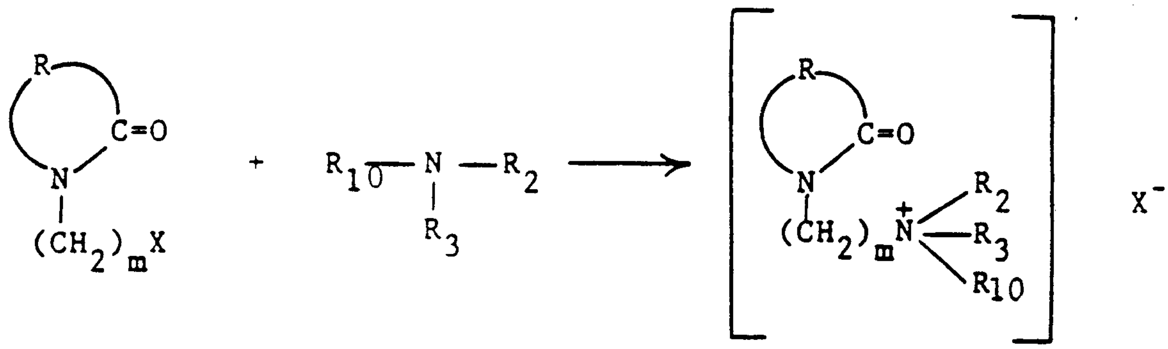

wherein m, R, $R_{10}$, $R_2$, $R_3$ and $X^-$ are as defined above and X is chloro, bromo or iodo.

Preferred tertiary amine coreactants employed in the reaction of group D include reactive methyl and ethyl tertiary amines having one or two $C_8$ to $C_{22}$ alkyl groups, for example N-methyl-N,N-didecyl amine, N,N-dimethyl-N-octadecyl amine, N,N-dimethyl-N-dodecyl amine, N,N-dimethyl-N-hexadecyl amine, N,N-dimethyl-N-eicosyl amine, and N-ethyl-N,N-dihexadecyl amine. However, also included in the scope of this invention are reactive amines wherein a $C_8$ to $C_{30}$ alkyl group is itself substituted with alkoxy of 1 to 4 carbon atoms, phenyl, benzyl, amido, can be employed, as well as tertiary amines having 1 to 3 aryl groups, eg. phenyl, benzyl, tolyl, xylyl, etc., such as triphenyl amine, dimethyl tolyl amine, ethyl di(phenyl) amine; methyl di(nonylphenyl) amine, dimethyl chlorophenyl amine and other halo substituted phenyl amines. Tri- $C_8$ to $C_{22}$ alkyl amines can also be employed in the reaction, examples of which include trihexadecyl amine, trioctadecyl amine, tridodecyl amine, tridecyl amine, etc. Also included are heterocyclic tertiary amines such as morpholine, imidazoline, piperazine, pyridine etc.

The most preferred amine coreactants of this invention are those defined by the formula

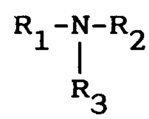

wherein $R_1$ is methyl, $R_2$ is methyl or $C_8$ to $C_{22}$ alkyl and $R_3$ is $C_8$ to $C_{22}$ alkyl.

The above processes are effected by reacting the haloalkyl lactam and the tertiary amine coreactant at a temperature between 25° and 150°C, preferably between 60° and 100°C, under a pressure of from 0 to 0.34 MPa (50 psig), preferably atmospheric pressure, for a period up to about 10 hours, although usually not more than 5 hours is required to complete the reaction.

The process is effected by reacting the haloalkyl lactam and the nitrogen heterocyclic amino coreactant at a temperature between 25° and 120°C, preferably between 60° and 100°C, under a pressure of from 0 to 0.34 MPa (50 psig), preferably atmospheric pressure, for a period up to about 10 hours, usually not more than 5 hours is required to complete the reaction. From the above equations, it is seen that most often stoichometric amounts of haloalkyl lactam and amine are used in the reaction. However, an excess of one or the other of the components is practicable in the process. Generally, for economic considerations, and where the coreactant contains a single quaternizable site, a mole ratio of between 1:1.5 and 1.5:1 is employed; although, a slight excess of the tertiary amino coreactant to insure complete reaction of the lactam is recommended. Accordingly, the most preferred mole ratio of lactam to amine is 1:1.01 - 1:1.03.

It is to be understood, however, that in instances where more than one quaternizable site is present in the coreactant, the molar amount of the haloalkyl lactam can be increased accordingly, depending on the degree of quaternization desired.

It is recommended that the haloalkyl lactam be added gradually or dropwise to the amine at the beginning of the ensuing exothermic reaction. Generally, at the completion of the reaction, a solid product is formed and recovered. Since the reaction is quantitative, the product can be used as is or, when a slight excess of the amine is employed, it can be neutralized with a weak acid such as acetic, lactic or citric acid.

For incorporating into a standard formulation of shampoo, cream rinse, hand or body lotion, textile treating solution etc., the present product is dissolved in an inert solvent such as water, propylene glycol,

ethanol, etc., and the solution in the desired amount is mixed into the formulation to provide a homogeneous liquid, gel, cream or lotion. Incorporation of the present product is usually affected at room temperature under atmospheric pressure and requires no special formulating technique. However, for certain formulations incorporation of the present product can be effected at temperatures up to about 85°C. Amphoteric-containing shampoo formulations are best prepared by initially preparing an aqueous solution of the quaternized product and the amphoteric surfactant and then adding the solution to the shampoo formulation.

Having generally described the invention, reference is now had to the accompanying examples which set forth preferred embodiments, but which are not to be construed as limiting to the scope of the invention as more broadly set forth above and in the appended claims.

The preparations and testing of the various compounds of this invention are based on the individual subspecies A-D set forth above and are separately reported below. However, it is found that all of the compounds within the scope of this invention share a community of properties and uses and can be employed interchangeably.

GROUP A

EXAMPLE I

To a 1 liter, 4-neck flask equipped with mechanical stirrer, reflux condenser, thermometer, and dropping funnel is added N,N-dimethyl-N-(3-octadecanlamido propyl) amine (0.505 mole) which is heated with stirring to 70°C. under $N_2$ blanket after which the heating source is removed and N-chloromethyl-2-pyrrolidone (0.5 mole) is added to the amine dropwise over a period of 25 minutes. An exothermic reaction ensues and is controlled at 100°C. by the rate of addition of N-chloromethyl-2-pyrrolidone. The reaction mixture changes from a liquid to a paste during the addition of N-chloromethyl-2-pyrrolidone and finally to a solid on completion of the reaction. The yield of N,N-dimethyl-N-[(2-pyrrolidonyl)methyl]-N-(3-octadecanamidopropyl) ammonium chloride, m.p. 45-50°C. is quantitative; the content of the quaternary product being determined by titration. (Mercuric Acetate method as described by Sidney Siggia, "Quantitative Organic Analysis via Functional Group", 1963, 3d Ed., John Wiley & Sons, pages 552-554).

EXAMPLE II

The reaction of Example I was repeated except that the amine used was N,N-dimethyl-N-(3-docosanamidopropyl) amine (at a 2% molar excess with respect to N-chloromethyl-2-pyrrolidone). The product, N,N-dimethyl-N-[(2-pyrrolidonyl)methyl]-N-(3-docosanamidopropyl) ammonium chloride, was recovered in quantitative yield.

EXAMPLE III

The reaction of Example II is repeated except that the amine used is N,N-dimethyl-N-(2-decanamidoethyl) amine. The corresponding N,N-dimethyl-N-[(2-pyrrolidinyl)methyl]-N-(2-decanamidoethyl) ammonium chloride, is recovered in quantitative yield.

EXAMPLE IV

The reaction of Example II is repeated except that the amine used is N-methyl-N,N-bis-(2-decanamidoethyl) amine. The corresponding N-methyl-N-[(2-pyrrolidonyl)methyl]-N,N-bis-(2-decanamidoethyl) ammonium chloride, is recovered in quantitative yield.

EXAMPLE V

The reaction of Example II is repeated except that the amine used is N-hexadecyl-N-methyl-N-(4-octanamidobutyl) amine. The corresponding N-hexadecyl-N-methyl-N-[(2-pyrrolidonyl)methyl]-N-(4-octanamidobutyl) ammonium chloride, is recovered in quantitative yield.

EXAMPLE VI

The reaction of Example II is repeated except that the amine used is N-(hydroxypropyl)-N-methyl-N-(2-tetradecanamidoethyl) amine. The corresponding N-(hydroxypropyl)-N-methyl-N-[(2-pyrrolidonyl)methyl]-N-(2-tetradecanamidoethyl) ammonium chloride, is recovered in quantitative yield.

EXAMPLE VII

The reaction of Example II is repeated except that the amine used is N,N-dioctadecyl-N-(3-hexanamidopropyl) amine. The corresponding N,N-dioctadecyl-N-[(2-pyrrolidonyl)methyl]-N-(3-hexanamidopropyl) ammonium chloride, is recovered in quantitative yield.

EXAMPLE VIII

The reaction of Example II is repeated except that the amine used is N,N-didecyl-N-(2-propionamidoethyl) amine. The product, N,N-didecyl-N-[(2-pyrrolidonyl)methyl]-N-(2-propionamidoethyl) ammonium chloride is recovered in quantitative yield.

EXAMPLE IX

The reaction of Example II is repeated except that the amine used was N-methyl-N,N-bis(3-undecanamidopropyl) amine. The product N-methyl-N-[(2-pyrrolidonyl)methyl]-N,N-bis(3-undecanamidopropyl) ammonium salt was obtained in quantitative yield.

EXAMPLE X

The reaction of Example II is repeated except that the amine used is N,N-dimethyl-N-[(2-octadecylcarbamoyl)phenyl] amine. The corresponding N,N-dimethyl-N-[(2-octadecylcarbamoyl)-phenyl]-N-[(2-pyrrolidonyl)methyl] ammonium chloride is recovered in high yield.

EXAMPLE XI

The reaction of Example II is repeated except that the amine used is N,N,N-tris-(pentadecanamidophenyl) amine. The corresponding N-[(2-pyrrolidonyl)methyl]-N,N,N-tris-(pentadecanamidophenyl) ammonium salt is obtained in high yield.

EXAMPLE XII

The reaction of Example II is repeated except that the amine used is N,N-dimethyl-N-(3-docosanamidopropyl) amine. The product, N,N-dimethyl-N-[(2-pyrrolidonyl)methyl]-N-(3-docosanamidopropyl) ammonium chloride, mp. 62-66 ° C., obtained in quantitative yield.

EXAMPLE XIII

The reaction of Example II is repeated except that the lactam used is N-bromomethyl-2-azacycloheptanone. A high yield of the product, N,N-dimethyl-N-[(3-azacycloheptanonyl)methyl] (3-docosanamidopropyl) ammonium bromide is recovered as a solid.

Other products described herein are prepared by the general procedure outlined above with the substitution of the desired 2-pyrrolidonyl-, 2-piperidonyl-, 2-azacycloheptanonyl-, 2-azacycloctanonyl-, 2-azacyclononanonyl-, or 2-azacyclodecanonyl- lactam reactant and/or the substitution of the desired tertiary amine.

Although all of the present quaternized compounds possess excellent surfactant properties, certain members are particularly recommended for incorporation into specific types of cosmetic formulations. Table I lists some of such uses for individual quaternized products within the scope of this invention, together with a brief description of the benefits derived from their incorporation.

Generally, the present products are added to the formulations in amounts between about 0.1 and about 5 wt. %, based on active ingredients. The quaternized compounds in Table I have the general formula:

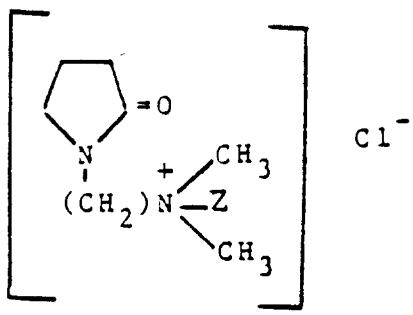

except No. 15 which has the formula:

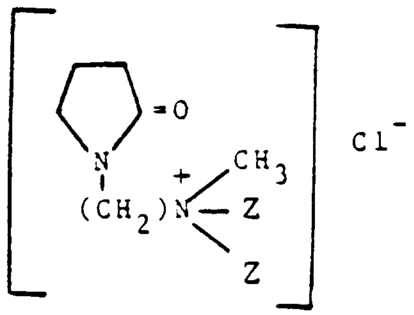

and No. 16 which has the formula:

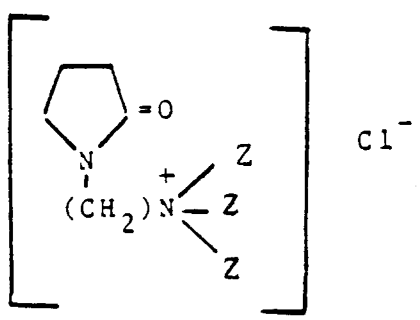

TABLE I

| No. | Compd where Z is | For Formulation In | Benefits Achieved |
|---|---|---|---|
| 1 | 3-octadecanamidopropyl or 4-(octadecylcarbamoyl) phenyl | cream rinse | improved wet/dry combing improved luster & shine |
| 2 | 3-octadecanamidopropyl or 4-hexadecanamidobutyl | hair conditioner | improved wet/dry combing overall conditioning & improved luster & shine |
| 3 | 3-hexadecanamidopropyl or 2-octadecanamidoethyl | blow-dry styling lotion | protection from heat, improved luster, shine, body & overall conditioning, preservation and styling |
| 4 | 3-octadecanamidopropyl or undecanamidophenyl | moisturizing lotion | facilitates rub-in, smoother feel & high compatability with other components |
| 5 | 3-octadecanamidopropyl or 4-octadecanamidophenyl | bubble bath | high compatability, smooth after-feel & viscosity enhancement |
| 6 | 2-hexadecanamidoethyl | mouthwash | good germidical properties clean, pleasant taste |
| 7 | 3-dodecanamidopropyl | syndet bar | high compatability & improved feel |
| 8 | 3-octadecanamidopropyl or hexadecylcarbamoylphenyl | after-sun lotion | high compatability, smoother feel & facilitates rub-in |

TABLE I

(Continued)

| | | | |
|---|---|---|---|
| 9 | 3-octadecan-amidopropyl | non-alcoholic mousse | conditioning, improved body, luster & shine, softer feel |
| 10 | 2-octadecan-amidoethyl | self-heating aerosol shave cream | high compatability, smoother skin feel, thermal stability & improved spreadability |
| 11 | 3-octadecan-amidopropyl or hexadecan-amidophenyl | mousse hand & body lotion | improved rub-in and smoother after-feel |
| 12 | Compound where Z is a mixture of 3-octadecan-amido propyl + 3-hexadecan-amidopropyl in n-dodecyl-2-pyrrolidone | conditioning shampoo | combined overall conditioning and cleaning, compatability with anionic components, improved wet/dry combing, luster & shine, viscosity enhancement & extra body |
| 13 | Compound where Z is 2-octa-decanamido ethyl in n-dodecyl-2-pyrrolidone | oily hair shampoo | "greaseless" conditioning, viscosity enhancement & improved wet/dry combability, shine & luster |
| 14 | Compound where Z is a mixture of 3-octa-decanamido-propyl and 2-hexadecanamido ethyl | hair mist conditioner | improved wet/dry combability, luster & shine, preservation and conditioning |
| | | hot oil treatment | improved conditioning, luster, shine, wet/dry combability, hair body & preservation |
| | | after shave balm | improved rub-in, skin conditioning & preservation and smoother skin feel |

TABLE I

(Continued)

| | | | |
|---|---|---|---|
| 15 | 3-octadecan-amidopropyl | conditoning hair spray | dry compatability, improved luster, shine and manageability. |
| 16 | dodecanamido-phenyl | sun blocking agent | filters out harmful rays good skin substantivity |
| 17 | octanamido-phenyl | sun tan agent | filters out harmful rays good skin substantivity |
| 18 | hexadecyl-carbamoyl-phenyl | sun tan agent and hair conditioner | filters out harmful rays good skin substantivity plus good hair combability |
| 19 | decanamido-tolyl | hair conditioner | improved hair combability, luster and shine |
| 20 | nonanamido-xylyl | sun tan agent | filters out harmful rays good skin substantivity |

Examples of specific formulations for the above uses achieving the benefits noted are presented as follows.

CREAM RINSE

| Ingredients | Parts by Weight |
|---|---|
| Compounds No. 1 in Table I | 2.0 |
| cetyl alcohol | 2.0 |
| emulsifying wax | 2.0 |
| citric acid | to pH 4 |
| deionized water | qs |
| fragrance | qs |
| preservative | qs |

HAIR CONDITIONER

| Ingredients | Parts by Weight |
|---|---|
| Compounds No. 2 in Table I | 4.0 |
| PEG-8 Distearate | 2.5 |
| mineral oil | 1.5 |
| lanolin alcohol | 1.0 |
| stearic acid | 1.0 |
| PPG-20 methyl glucose ether | 1.0 |
| hydrolized animal protein | 0.25 |
| citric acid | to pH 4 |
| deionized water | qs |
| preservative | qs |
| fragrance | qs |

BLOW DRY STYLING LOTION

| Ingredients | Parts by Weight |
|---|---|
| Compounds No. 3 in Table I | 1.5 |
| ethanol | 3.0 |
| polyquaternium* 11 | 2.0 |
| PEG-10 Castor oil | 0.2 |
| fragrance | 0.2 |
| phosphoric acid | to pH 6 |
| deionized water | qs |

* the quaternized ammonium polymer formed by reacting dimethyl sulfate and a copolymer of vinyl pyrrolidone and dimethylamino methylacrylate

CONDITIONING HAIR SPRAY

| Ingredients | Parts by Weight |
|---|---|
| Compound No. 15 in Table I | 0.6 |
| ethanol | 75.0 |
| ethyl ester of PVM/MA** copolymer | 4.1 |
| 2-amino-2-methyl-1-propanol 99% | 0.1 |
| fragrance | 0.2 |
| propellant | 20.0 |

** vinyl methyl ether/maleic anhydride

CONDITIONING SHAMPOO

| Ingredients | Parts by Weight |
|---|---|
| Compound mixture of No. 12 in Table I | |
| Z is 3-hexadecanamidopropyl | 1.5 |
| Z is 3-octadecanamidopropyl | 1.5 |
| N-dodecyl-2-pyrrolidone | 0.6 |
| polyquaternium 11 | 0.5 |
| sodium laureth-4-phosphate | 0.8 |
| ammonium lauryl sulfate | 40.0 |
| silk protein | 0.25 |
| tetrasodium ethylenediamine tetra-acetic acid | 0.2 |
| deionized water | qs |
| colorant | qs |
| fragrance | qs |

MOISTURIZING LOTION

| Ingredients | Parts by Weight |
|---|---|
| Compounds No. 4 in Table I | 2.0 |
| mineral oil 70 CTS | 2.0 |
| stearic acid | 3.0 |
| emulsifying wax | 3.0 |
| Dimethicone* 200 CTS | 1.5 |
| Carbomer 934** | 0.15 |
| Oleth - 20*** | 1.0 |
| triethanolamine 98% | 1.0 |
| deionized water | qs |
| preservative | qs |
| fragrance | qs |

* a mixture of methylated siloxane polymers end - blocked with trimethyl siloxy units (dimethylpolysiloxane)
** cross - linked polymer of acrylic acid
*** PEG ether of oleyl alcohol

BUBBLE BATH

| Ingredients | Parts by Weight |
|---|---|
| Compounds No. 5 in Table I | 3.0 |
| ammonium nonoynol-4-sulfate | 30.0 |
| sodium cocoyl isothionate | 10.0 |
| cocamidopropyl hydroxysultaine | 10.0 |
| cocamide diethanolamide | 6.0 |
| sodium methyl cocyl taurate | 20.0 |
| aloe vera gel | 1.0 |
| coconut oil | 1.0 |
| glycol stearate | 1.0 |
| deionized water | qs |
| preservative | qs |
| colorant | qs |

SHAMPOO FOR OILY HAIR

| Ingredients | Parts by Weight |
|---|---|
| Compound No. 13 in Table I | 3.0 |
| n-dodecylpyrrolidone | 1.0 |
| tetrasodium ethylenediamine tetra-acetic acid | 0.2 |
| sodium lauryl sulfate | 20.0 |
| alpha-olefin sulfonate | 20.0 |
| polyquaternium 11 | 0.5 |
| deionized water | qs |
| preservative | qs |
| colorant | qs |
| fragrance | qs |
| added inorganic salts as desired for viscosity modification | |

MOUTHWASH

| Ingredients | Parts by Weight |
|---|---|
| Compound No. 6 in Table I | 0.05 |
| alcohol, 190° | 20.00 |
| thymol | 0.03 |
| glycerine | 10.00 |
| flavor | 2.0 |
| distilled water | qs |
| polysorbate 80 | 2.00 |

SYNDET BAR (Superfatted)

| Ingredients | Parts by Weight |
|---|---|
| Compound No. 7 in Table I | 0.5 |
| stearic acid, triple pressed | 32.00 |
| kettle soap | 9.80 |
| sodiumcocoyl isethionate | 49.00 |
| sodium methyl cocoyl taurate | 6.90 |
| citric acid, 50% aqueous | 0.60 |
| titanium dioxide | 0.20 |
| fragrance | 1.00 |

WATER RESISTANT EMOLLIENT AFTER SUN LOTION

| Ingredients | Parts by Weight |
|---|---|
| Compounds No. 8 in Table I | 3.0 |
| mink Oil, Light Fraction | 11.00 |
| glyceryl stearate, self emulsifying | 1.00 |
| stearic acid | 2.50 |
| mineral oil and lanolin alcohol | 2.00 |
| myristyl myristate | 3.000 |
| mineral oil | 10.00 |
| PVP/Eicosene copolymer | 2.00 |
| triethanolamine | 0.70 |
| sorbitol | 3.00 |
| hydroxyethylcellulose | 0.30 |
| distilled water | qs |
| preservative | qs |
| fragrance | qs |

NON-ALCOHOLIC CONDITIONING MOUSSE

| Ingredients | Parts by Weight |
|---|---|
| Compound No. 9 in Table I | 5.00 |
| PVP K-30 | 2.00 |
| Oleth-2O | 0.50 |
| fragrance | qs |
| deionized water | 77.50 |
| propellant A-46 | 15.00 |

SELF-HEATING AEROSOL SHAVING CREAM

Employed dual dispensing valve for metering oxidant from $H_2O_2$ container and reductant from aerosol can.

| Ingredients | Parts by Weight |
|---|---|
| Compound No. 10 in Table I | 2.00 |
| stripped coconut fatty acid | 1.10 |
| sorbitol | 10.00 |
| stearic acid | 4.20 |
| PEG-40 soritan peroleate | 2.00 |
| triethanolamine | 3.00 |
| potassium hydroxide | 1.00 |
| potassium sulfite | 9.00 |
| fragrance | 0.80 |
| butyrated hydroxy toluene (BHT) | 0.01 |
| butyrated hydroxy anisole (BHA) | 0.01 |
| deionized water | qs |
| propellant | qs |

HAIR MIST CONDITIONER (w/o added preservative)

| Ingredients | Parts by Weight |
|---|---|
| 50/50 Mixture No. 14 in Table I | 1.00 |
| propylene glycol dicaprylate/dicaprate copolymer | 0.30 |
| oleamidopropyl dimethylamine | 0.50 |
| deionized water | 98.2 |

CATIONIC MOUSSE HAND/BODY LOTION
(Used 85 Parts of the following formula to 15 parts propellant A-46)

| Ingredients | Parts by Weight |
|---|---|
| Compounds No. 11 in Table I | 0.50 |
| acetylated polyoxyethylene lanolin | 2.00 |
| ethoxylated lanolin alcohols | 1.00 |
| glyceryl stearate, self-emulsifying | 5.50 |
| cetyl alcohol | 1.50 |
| mineral oil, 70 CTS | 1.50 |
| stearyl alcohol | 1.50 |
| glycerin | 3.00 |
| isopropyl myristate | 4.00 |
| dimethicone, 100 CTS | 2.00 |
| water | qs |
| preservative | qs |
| fragrance | qs |

HOT OIL TREATMENT – (w/o added preservative)

| Ingredients | Parts by Weight |
|---|---|
| 50/50 mixture No. 14 in Table I | 1.50 |
| oleamidopropyl dimethyl amine | 1.00 |
| polyethylene glycol 6000 distearate | 2.00 |
| hexylene glycol | 4.00 |
| lactic acid, 88% | to pH 4.4 |
| color | qs |
| deionized water | qs |

AFTER SHAVE BALM

| Ingredients | Parts by Weight |
|---|---|
| 50/50 mixture No. 14 in Table I | 1.00 |
| Carbomer 941 | 0.20 |
| tetrasodium ethylene diamine tetraacetic acid | 0.10 |
| cetearyl alcohol* and polyethylene glycol ether of cetearyl alcohol | 2.50 |
| isopropyl myristate | 1.00 |
| Oleth – 20 | 1.00 |
| methyl gluceth 20 | 2.00 |
| triethanolamine, 98% | 0.20 |
| propylene glycol | 3.00 |
| SDA denatured alcohol | 7.50 |
| PVP/dimethylaminoethyl methacrylate | 7.00 |
| fragrance | 1.00 |
| distilled water | qs |

* 50/50 mixture of cetyl and stearyl alcohols

SUN BLOCK LOTION

| Ingredients | Parts by Weight |
|---|---|
| Compound No. 16 in Table I | 7.93 |
| Acetylated lanolin | 1.22 |
| Isooctyl acrylate/octadecyl acrylate/acrylic acid terpolymer (mol ratio = 40/40/20) | 3.05 |
| $C_{11-13}$ isoparaffin solvent | 9.15 |
| Isopropyl palmitate | 9.15 |
| Isostearyl alcohol | 4.27 |
| Carnation mineral oil | 4.27 |
| Deionized water | 60.96 |

SKIN TANNING LOTION

| Ingredients | Parts by Weight |
|---|---|
| Compound No. 19 in Table I | 5.0 |
| Cetyl alcohol, NF | 3.6 |
| Coceth-6 | 0.9 |
| Hydroxyethylcellulose (Cellosize QP-100 M; Union Carbide Corp.) | 0.6 |
| PEG-8 (humectant) | 1.0 |
| Perfume | 0.4 |
| Titanium dioxide | 0.15 |
| Ethanol | 53.5 |
| Water (balance) 35% | |

The above examples are representative or preferred embodiments of the present invention; however, it will be understood that other species of instant quaternized lactams can be substituted in the above formulations to provide the benefits indicated. Also, in the preparation of the quaternized lactams described in Examples I-XIII, other lactam and/or tertiary amine reactants described herein can be substituted to provide the corresponding quaternized products which are also included within the scope of this invention. Particularly recommended among these substituted species are the halomethyl-2-piperidonyl reactants and the other tertiary amido amine reactants which also provide useful bactericidal and viscosity enhancing properties.

GROUP B

EXAMPLE XIV

To a 1 liter, 4-neck flask equipped with mechanical stirrer, reflux condenser, thermometer, and dropping funnel is added N,N-dimethyl-N-[3-(octadecyloxy)propyl] amine (0.505 mole) which is heated with stirring to 70°C. under $N_2$ blanket after which the heating source was removed and N-chloromethyl-2-pyrrolidone (0.5 mole) is added to the ether amine dropwise over a period of 25 minutes. An exothermic reaction ensues and is controlled at 100°C. by the rate of addition of N-chloromethyl-2-pyrrolidone. The yield of lqiuid N,N-dimethyl-N-[3-(octadecyloxy)propyl]-N-[(2-pyrrolidonyl)methyl] ammonium chloride is quantitative which is determined by titration. (Mercuric Acetate method as described by Sidney Siggia, "Quantitative Organic Analysis via Functional Group", 1963, 3d Ed., John Wiley & Sons, pages 552-554).

EXAMPLE XV

The reaction of Example XIV is repeated except that the ether amine used is N,N-dimethyl-N-[2-(hexadecyloxy)ethyl] amine (2% molar excess with respect to N-chloromethyl-2- pyrrolidone). The product, N,N-dimethyl-N-[2-(hexadecyloxy)ethyl]-N-[(2-pyrrolidonyl)-methyl] ammonium chloride is neutralized with acetic acid and recovered in quantitative yield.

EXAMPLE XVI

The reaction of Example XV is repeated except that the ether amine used is N,N-dimethyl-N-[4-(tetradecyloxy)butyl] amine. The corresponding N,N-dimethyl-N-[4-(tetradecyloxy)butyl]-N-[(2-pyrrolidonyl) methyl] ammonium chloride is recovered in quantitative yield.

EXAMPLE XVII

The reaction of Example XV is repeated except that the ether amine used is N,N-dimethyl-N-[3-(dodecyloxy)propyl] amine. The corresponding N,N-dimethyl-N-[3-dodecyloxy)propyl]-N-[(2-pyrrolidonyl)methyl] ammonium chloride is recovered in quantitative yield.

EXAMPLE XVIII

The reaction of Example XV is repeated except that the ether amine used is N,N – dimethyl – N – [3 – (decyloxy) propyl] amine. The corresponding N,N – dimethyl – N – [3 – decyloxy)propyl] – N – [(2 – pyr – rolidonyl) methyl] ammonium chloride is recovered in quantitative yield.

EXAMPLE XIX

The reaction of Example XV is repeated except that the ether amine used is N – methyl – N – octadecyl – N – [3 – (octyloxy)propyl] amine. The corresponding N – methyl – N – octadecyl – N – [3 – (octyloxy)propyl] – N – [(2 – pyrrolidonyl) methyl] ammonium chloride is recovered in quantitative yield.

EXAMPLE XX

The reaction of Example XV is repeated except that the ether amine used is N,N – [3 – di – (octadecyloxy)propyl] – N – methyl amine. The corresponding N – methyl – N,N – [3 – di(octadecyloxy) – propyl] – N – [(2 – pyrrolidonyl) methyl] ammonium chloride is recovered in quantitative yield.

EXAMPLE XXI

The reaction of Example XV is repeated except that the ether amine used is N,N – didecyl – N – [2 – (methyloxy) ethyl] amine. The product, N – [2 – (methyloxy)ethyl] – N,N – didecyl – N – [(2 – pyrrolidonyl) methyl] ammonium chloride is obtained in quantitative yield.

EXAMPLE XXII

The reaction of Example XV is repeated except that the ether amine used is N,N – dimethyl – N – [3 – (tridecyloxy)propyl] amine. The product, N,N – dimethyl – N – [(2 – pyrrolidonyl)methyl] – N – [3 – (tridecyloxy) propyl] ammonium chloride is obtained in quantitative yield.

EXAMPLE XXIII

The reaction of Example XV is repeated except that the lactam used is N – bromomethyl – 2 – caprolactam, the N,N – dimethyl – N – [2 – (hexadecyloxy)ethyl] – N – [(2 – azacycloheptanonyl)methyl] am – monium bromide is recovered as a solid product.

Other lactam containing products described herein are prepared by the general procedure outlined above with the substitution of the desired 2 – pyrrolidonyl – , 2 – piperidonyl – , 2 – azacycloheptanonyl – , 2 – azacycloctanonyl – , 2 – azacyclononanoyl – , or 2 – azacyclodecanonyl – lactam reactant and/or the sub – stitution of the desired tertiary ether amine.

Although all of the present quaternized compounds possess excellent surfactant properties, certain members are particularly recommended for incorporation into specific types of cosmetic formulations. Table II lists some of such uses for individual quaternized products within the scope of this invention, together with a brief description of the benefits derived from their incorporation.

Generally, the present products are added to the formulations in amounts between about 0.1 and about 5 wt. %, based on active ingredients. The quaternized compounds in Table II have the general formula:

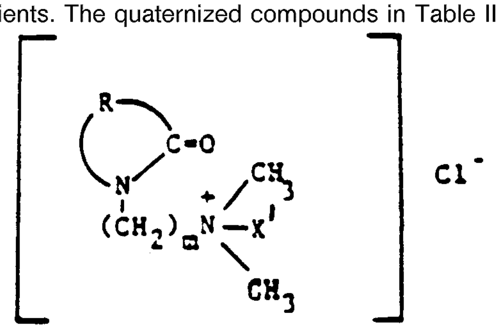

except No. 16 which has the formula:

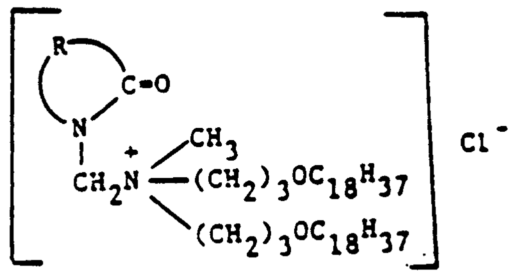

TABLE II

| No. | Compd where X' is | For Formulation In | Benefits Achieved |
|---|---|---|---|
| 1 | [3-(octadecyloxy)propyl] | cream rinse | improved wet/dry combing improved luster & shine |
| 2 | [4-(octadecyloxy)butyl] or [3-(15-octadecen-1-yloxy)propyl] | hair conditioner | improved wet/dry combing overall conditioning & improved luster & shine |
| 3 | [2-(hexadecyloxy)ethyl] | blow-dry styling lotion | protection from heat, improved luster, shine, body & overall conditioning, preservation and styling |
| 4 | [3-(alkyloxy) propyl] where alkyl is a mixture of $C_{12}$ to $C_{15}$ derived from NEODOL 25 | moisturizing lotion | facilitates rub-in, smoother feel & high compatability with other components |
| 5 | [3-(octadecyloxy)propyl] or [5,6-(diethyl) heptyl-1-yloxy)propyl] | bubble bath | high compatability, smooth after-feel & viscosity enhancement |
| 6 | [3-(dodecyloxy)propyl] | mouthwash | good germidical properties clean, pleasant taste |
| 7 | [3-(octadecyloxy) propyl] | syndet bar | high compatability & improved feel |

TABLE II

(Continued)

| | | | |
|---|---|---|---|
| 8 | [4-(octadecyloxy) butyl] or [4-(11-dodecen-1-yloxy)butyl] | after-sun lotion | high compatability, smoother feel & facilitates rub-in |
| 9 | [2-(octadecyloxy) ethyl] | non-alcoholic mousse | conditioning, improved body, luster & shine, softer feel |
| 10 | [2-(octadecyloxy) methyl] | self-heating aerosol shave cream | high compatability, smoother skin feel, thermal stability & improved spreadability |
| 11 | [4-(octadecyloxy) butyl] | mousse hand & body lotion | improved rub-in and smoother after-feel |
| 12 | [3-(alkoxy) propyl] in which alkyl is a 50/50 mixture of $C_{12}$, $C_{14}$ and $C_{16}$ | mousse hand & body lotion | improved rub-in and smoother after-feel |
| 13 | compd. where X' is [3 (octadecyloxy) propyl] + compd. where X' is [3-(hexadecyloxy) propyl] combined in n-dodecyl-2-pyrrolidone | conditioning shampoo | combined overall conditioning and cleaning, compatability with anionic components, improved wet/dry combing, luster & shine, viscosity enhancement & extra body |

TABLE II

(Continued)

| | | | |
|---|---|---|---|
| 14 | compd. where X' is [4-(octadecyloxy) butyl] in n-dodecyl-2-pyrrolidone | oily hair shampoo | "greaseless" conditioning, viscosity enhancement & improved wet/dry combability, shine & luster |
| 15 | compd. where X' is [4-octadecyloxy) butyl] + compd. where X' is [4-(hexadecyloxy) butyl] | hair mist conditioner | improved wet/dry combability, luster & shine, preservation and conditioning |
| | | hot oil treatment | improved conditioning, luster, shine, wet/dry combability, hair body & preservation |
| | | after shave balm | improved rub-in, skin conditioning & preservation and smoother skin feel |
| 16 | [3-di(octadecyloxy) propyl] | conditoning hair spray | dry compatability, improved luster, shine and manageability. |

Examples of specific formulations for the above uses achieving the benefits noted are presented as follows.

EXAMPLE XXIV

CREAM RINSE

| Ingredients | Parts by Weight |
|---|---|
| Compound No. 1 in Table II | 2.0 |
| cetyl alcohol | 2.0 |
| emulsifying wax | 2.0 |
| citric acid | to pH 4 |
| deionized water | qs |
| fragrance | qs |
| preservative | qs |

HAIR CONDITIONER

| Ingredients | Parts by Weight |
|---|---|
| Compounds No. 2 in Table II | 4.0 |
| PEG-8 Distearate | 2.5 |
| mineral oil | 1.5 |
| lanolin alcohol | 1.0 |
| stearic acid | 1.0 |
| PPG-20 methyl glucose ether | 1.0 |
| hydrolized animal protein | 0.25 |
| citric acid | to pH 4 |
| deionized water | qs |
| preservative | qs |
| fragrance | qs |

BLOW DRY STYLING LOTION

| Ingredients | Parts by Weight |
|---|---|
| Compound No. 3 in Table II | 1.5 |
| ethanol | 3.0 |
| polyquaternium* 11 | 2.0 |
| PEG-10 Castor oil | 0.2 |
| fragrance | 0.2 |
| phosphoric acid | to pH 6 |
| deionized water | qs |

* the quaternized ammonium polymer formed by reacting dimethyl sulfate and a copolymer of vinyl pyrrolidone and dimethylamino methylacrylate

CONDITIONING HAIR SPRAY

| Ingredients | Parts by Weight |
|---|---|
| Compound No. 16 in Table II | 0.6 |
| ethanol | 75.0 |
| ethyl ester of PVM/MA** copolymer | 4.1 |
| 2-amino-2-methyl-1-propanol 99% | 0.1 |
| fragrance | 0.2 |
| propellant | 20.0 |

** vinyl methyl ether/maleic anhydride

CONDITIONING SHAMPOO

| Ingredients | Parts by Weight |
|---|---|
| Compounds of the type in Table II where X' is a mixture of | |
| [3-(dodecyloxy)propyl] and | 1.5 |
| [3-(tetradecyloxy)propyl] | 1.5 |
| N-dodecyl-2-pyrrolidone | 0.6 |
| polyquaternium 11 | 0.5 |
| sodium laureth-4-phosphate | 0.8 |
| ammonium lauryl sulfate | 40.0 |
| silk protein | 0.25 |
| tetrasodium ethylenediamine tetra-acetic acid | 0.2 |
| deionized water | qs |
| colorant | qs |
| fragrance | qs |

MOISTURIZING LOTION

| Ingredients | Parts by Weight |
|---|---|
| Compound No. 4 in Table II | 2.0 |
| mineral oil 70 CTS | 2.0 |
| stearic acid | 3.0 |
| emulsifying wax | 3.0 |
| Dimethicone* 200 CTS | 1.5 |
| Carbomer 934** | 0.15 |
| Oleth - 20*** | 1.0 |
| triethanolamine 98% | 1.0 |
| deionized water | qs |
| preservative | qs |
| fragrance | qs |

* a mixture of methylated siloxane polymers end - blocked with trimethyl siloxy units (dimethylpolysiloxane)

** cross - linked polymer of acrylic acid

*** PEG ether of oleyl alcohol

BUBBLE BATH

| Ingredients | Parts by Weight |
|---|---|
| Compounds No. 5 in Table II | 3.0 |
| ammonium nonoynol-4-sulfate | 30.0 |
| sodium cocoyl isothionate | 10.0 |
| cocamidopropyl hydroxysultaine | 10.0 |
| cocamide diethanolamide | 6.0 |
| sodium methyl cocyl taurate | 20.0 |
| aloe vera gel | 1.0 |
| coconut oil | 1.0 |
| glycol stearate | 1.0 |
| deionized water | qs |
| preservative | qs |
| colorant | qs |

SHAMPOO FOR OILY HAIR

| Ingredients | Parts by Weight |
|---|---|
| Compound No. 14 in Table II | 3.0 |
| n-dodecylpyrrolidone | 1.0 |
| tetrasodium ethylenediamine tetra-acetic acid | 0.2 |
| sodium lauryl sulfate | 20.0 |
| alpha-olefin sulfonate | 20.0 |
| polyquaternium 11 | 0.5 |
| deionized water | qs |
| preservative | qs |
| colorant | qs |
| fragrance | qs |
| added inorganic salts as desired for viscosity modification | |

MOUTHWASH

| Ingredients | Parts by Weight |
|---|---|
| Compound No. 6 in Table II | 0.05 |
| alcohol, 190° | 20.00 |
| thymol | 0.03 |
| glycerine | 10.00 |
| flavor | 2.0 |
| distilled water | qs |
| polysorbate 80 | 2.00 |

SYNDET BAR (Superfatted)

| Ingredients | Parts by Weight |
|---|---|
| Compound No. 7 in Table II | 0.5 |
| stearic acid, triple pressed | 32.00 |
| kettle soap | 9.80 |
| sodiumcocoyl isethionate | 49.00 |
| sodium methyl cocoyl taurate | 6.90 |
| citric acid, 50% aqueous | 0.60 |
| titanium dioxide | 0.20 |
| fragrance | 1.00 |

WATER RESISTANT EMOLLIENT AFTER SUN LOTION

| Ingredients | Parts by Weight |
|---|---|
| Compounds No. 8 in Table II | 3.0 |
| mink Oil, Light Fraction | 11.00 |
| glyceryl stearate, self emulsifying | 1.00 |
| stearic acid | 2.50 |
| mineral oil and lanolin alcohol | 2.00 |
| myristyl myristate | 3.000 |
| mineral oil | 10.00 |
| PVP/Eicosene copolymer | 2.00 |
| triethanolamine | 0.70 |
| sorbitol | 3.00 |
| hydroxyethylcellulose | 0.30 |
| distilled water | qs |
| preservative | qs |
| fragrance | qs |

NON-ALCOHOLIC CONDITIONING MOUSSE

| Ingredients | Parts by Weight |
|---|---|
| Compound No. 9 in Table II | 5.00 |
| PVP K-30 | 2.00 |
| Oleth-2O | 0.50 |
| fragrance | qs |
| deionized water | 77.50 |
| propellant A-46 | 15.00 |

SELF-HEATING AEROSOL SHAVING CREAM

Employed dual dispensing valve for metering oxidant from $H_2O_2$ container and reductant from aerosol can.

| Ingredients . | Parts by Weight |
|---|---|
| Compound No. 10 in Table II | 2.00 |
| stripped coconut fatty acid | 1.10 |
| sorbitol | 10.00 |
| stearic acid | 4.20 |
| PEG - 40 soritan peroleate | 2.00 |
| triethanolamine | 3.00 |
| potassium hydroxide | 1.00 |
| potassium sulfite | 9.00 |
| fragrance | 0.80 |
| butyrated hydroxy toluene (BHT) | 0.01 |
| butyrated hydroxy anisole (BHA) | 0.01 |
| deionized water | qs |
| propellant | qs |

HAIR MIST CONDITIONER (w/o added preservative)

| Ingredients | Parts by Weight |
|---|---|
| 50/50 Mixture No. 15 in Table II | 1.00 |
| propylene glycol dicaprylate/dicaprate copolymer | 0.30 |
| oleamidopropyl dimethylamine | 0.50 |
| deionized water | 98.2 |

CATIONIC MOUSSE HAND/BODY LOTION
(Used 85 Parts of the following formula to 15 parts propellant A - 46)

| Ingredients | Parts by Weight |
|---|---|
| Compound No. 11 in Table II | 0.50 |
| acetylated polyoxyethylene lanolin | 2.00 |
| ethoxylated lanolin alcohols | 1.00 |
| glyceryl stearate, self - emulsifying | 5.50 |
| cetyl alcohol | 1.50 |
| mineral oil, 70 CTS | 1.50 |
| stearyl alcohol | 1.50 |
| glycerin | 3.00 |
| isopropyl myristate | 4.00 |
| dimethicone, 100 CTS | 2.00 |
| water | qs |
| preservative | qs |
| fragrance | qs |

HOT OIL TREATMENT – (w/o added preservative)

| Ingredients | Parts by Weight |
|---|---|
| 50/50 mixture No. 15 in Table II | 1.50 |
| oleamidopropyl dimethyl amine | 1.00 |
| polyethylene glycol 6000 distearate | 2.00 |
| hexylene glycol | 4.00 |
| lactic acid, 88% | to pH 4.4 |
| color | qs |
| deionized water | qs |

AFTER SHAVE BALM

| Ingredients | Parts by Weight |
|---|---|
| 50/50 mixture No. 15 in Table II | 1.00 |
| Carbomer 941 | 0.20 |
| tetrasodium ethylene diamine tetraacetic acid | 0.10 |
| cetearyl alcohol* and polyethylene glycol ether of cetearyl alcohol | 2.50 |
| isopropyl myristate | 1.00 |
| Oleth-20 | 1.00 |
| methyl gluceth 20 | 2.00 |
| triethanolamine, 98% | 0.20 |
| propylene glycol | 3.00 |
| SDA denatured alcohol | 7.50 |
| PVP/dimethylaminoethyl methacrylate | 7.00 |
| fragrance | 1.00 |
| distilled water | qs |

* 50/50 mixture of cetyl and stearyl alcohols

The above examples are representative or preferred embodiments of the present invention; however, it will be understood that other species of instant quaternized lactams can be substituted in the above formulations to provide the benefits indicated. Also, in the preparation of the quaternized lactams described in Examples XIV-XXIII, other lactam and/or tertiary amino ethers described herein can be substituted to provide the corresponding quaternized products which are also included within the scope of this invention. Particularly recommended among these substituted species are the halomethyl-2-caprolactam reactants and the alkyl amidoalkyl-substituted or hydroxyalkyl-substituted tertiary ether amine reactants which also provide useful bactericidal and viscosity enhancing properties.

GROUP C

EXAMPLE XXV

To a 1 liter, 4-neck flask equipped with mechanical stirrer, reflux condenser, thermometer, and dropping funnel was added pyridine (0.505 mole) which was heated with stirring to 70 °C. under $N_2$ blanket after which the heating source was removed and N-chloromethyl-2-pyrrolidone (0.5 mole) was added to the amine dropwise over a period of 25 minutes. An exothermic reaction ensued and was controlled at 100 °C. by the rate of addition of N-chloromethyl-2- pyrrolidone. The reaction mixture changed from a liquid to a paste during the addition of N-chloromethyl-2-pyrrolidone and finally to a solid (powder) on completion of the reaction. The yield of N-[(2-pyrrolidonyl)methyl] pyridinium chloride, m.p. 160-162 °C., was quantitative. The content of the quaternary compound was determined by titration. (Mercuric Acetate method as described by Sidney Siggia, "Quantitative Organic Analysis via Functional Group", 1963, 3d Ed., John Wiley & Sons, pages 552-554).

EXAMPLE XXVI

The reaction of Example XXV was repeated except that the amine used was N-methyl-morpholine (2% molar excess with respect to N-chloromethyl-2-pyrrolidone). The product, N-ethyl-N-[(2-pyrrolidonyl)methyl] morpholinium chloride was recovered as a paste in quantitative yield.

EXAMPLE XXVII

The process of Example XXV was repeated except that the amine used was 3-octadecyl oxazolidine. The yield of N-octadecyl-N-[(2-pyrrolidonyl)methyl] oxazolidinium chloride is quantitative.

EXAMPLE XXVIII

The process of Example XXV was repeated except that the amine used was 5-methyl-isoxazolidine. The yield of N-hexadecyl-5-methylN-[(2-pyrrolidonyl)methyl] oxazolidinium chloride is essentially quantitative.

EXAMPLE XXIX

The process of Example XXV was repeated except that the amine used was 1-(2-hydroxyethyl)-2-heptadecyl-2-imidazoline. The yield of 1-(2-hydroxyethyl)-2-heptadecyl-1(or 3)-[(2-pyrrolidonyl) methyl] 2-imidazolinium chloride is quantitative.

EXAMPLE XXX

The process of Example XXV was repeated except that the amine used was 1-(2-hydroxyethyl)-2-undecylimidazoline. The yield of 1-(2-hydroxyethyl)-2-undecyl-1(or 3)-[(2-pyrrolidonyl) methyl] imidazolinium chloride is quantitative.

EXAMPLE XXXI

The process of Example XXV was repeated except that the amine used was 3-hexadecyl-pyridine. The yield of 3-hexadecyl-N-[(2-pyrrolidonyl)methyl] pyridinium chloride.

EXAMPLE XXXII

The process of Example XXV was repeated except that the amine used was N-octadecyl-piperidine. The yield of N-octadecyl-N-[(2-pyrrolidonyl)methyl] piperidinium chloride is quantitative.

EXAMPLE XXXIII

The process of Example XXV was repeated except that the amine used was N-octadecyl-morpholine. The yield of N-octadecyl-N-[(2-pyrrolidonyl)methyl] morpholinium chloride is quantitative.

EXAMPLE XXXIV

The process of Example XXXIII was repeated except that N-chloromethyl caprolactam was used instead of N-chloromethyl-2-pyrrolidone. The yield of N-octadecyl-N-[(2-azacycloheptanonyl)-methyl] morpholinium chloride is quantitative.

EXAMPLE XXXV

The process of Example XXV was repeated except that the amine used was N-dodecyl-indoline. The yield of N-dodecyl-N-[(2-pyrrolidonyl)methyl] indolinium chloride is quantitative.

EXAMPLE XXXVI

The process of Example XXV is repeated except that N-octadecyl thiazine is substituted for pyridine. The yield of N-octadecyl-N-[(2-pyrrolidonyl)methyl] thiazinium chloride is quantitative.

EXAMPLE XXXVII

The process of Example XXV is repeated except that N,N'-diethyl diazacyclotetradecane is substituted for pyridine and the amount of N-chloromethyl-2-pyrrolidone reactant is doubled. The yield of N,N'-diethyl-N,N'-bis-[(2-pyrrolidonyl)methyl] diazacyclotetradicanium chloride product is quantitative.

EXAMPLE XXXVIII

Although all of the present quaternized compounds possess excellent surfactant properties, certain members are particularly recommended for incorporation into specific types of cosmetic formulations. Table III lists some of such uses for individual quaternized products within the scope of this invention, together with a brief description of the benefits derived from their incorporation.

Generally, the present products are added to the formulations in amounts between about 0.1 and about 5 wt. %, based on active ingredients. The quaternized compounds in Table III have the general formula:

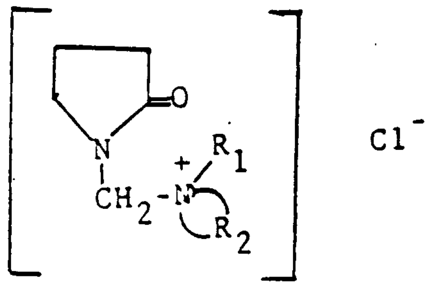

TABLE III

| No. | Compound where $R_2$ is/$R_1$ is | For Formulation In | Benefits Achieved |
|---|---|---|---|
| 1 | $-(CH_2)_7-$/ $-C_2H_4OH$ | cream rinse | improved wet/dry combing-luster-softness |
| 2 | $-(CH_2)_4-$/ $-C_6H_4-C_8H_{17}$ | hair conditioner | improved wet/dry combing overall conditioning & increased luster & shine |
| 3. | 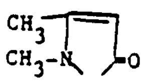 / $-C_6H_5$ (derived from antipyrine) | blow-dry styling & moisturizing lotion | protection from heat-increased luster, shine, body & overall conditioning |
| 4. | 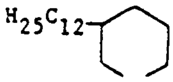 / $-CH_3$ (derived from 3-dodecyl piperidine) | bubble bath & textile softener | high compatability, smooth hand & viscosity enhancement |
| 5. | 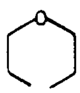 / $-C_{18}H_{37}$ (derived from N-octadecyl morpholine) | syndet bar & after-sun lotion | improved moisturizing - high compatability & easy rubb-in |
| 6. | 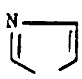 / $-C_{14}H_{29}$ (derived from N-tetradecyl imidazole) | non-alcoholic mousse | conditioning-increased body, luster & shine |
| 7. | 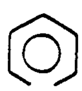 / -* (derived from pyridine) | aerosol shave creams | high compatability-improved spreadability skin smoothing |
| 8. | 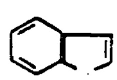 / $-C_{12}H_{25}$ (derived from N-dodecyl indole) | mousse hand & body lotion | improved rubb-in & smoother after-feel |
| 9. | 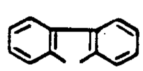 /$-C_{16}H_{33}$ (derived from N-hexadecyl carbazole) | moisturizing lotion | high compatability-good skin substantivity and penetration |

TABLE III

(continued))

| | | | |
|---|---|---|---|
| 10. | 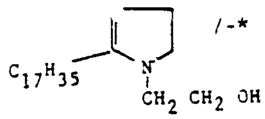 /-* (derived from N-hydroxy-ethyl-2-heptadecyl imidazoline) | hair conditioner | improved combability, luster & shine |
| 11 | $-CH=CH-O-CH=CH-$/ $-C_{12}H_{25}$ (derived from N-dodecyl oxazine) | conditioning shampoo | overall conditioning & cleaning- compatability with anionic components- viscosity enhancing |
| 12 | $-(CH_2)_9-$/ $-C_6H_4(CH_3)$ | oily hair shampoo | greaseless conditioning- viscosity building- improved wet/dry combability |
| 13 | $-(CH_2)_4-$/ $-C_6H_3(CH_3)_2$ | hair mist conditioner | improved wet/dry combing, luster & shine |
| 14. | 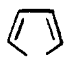 /$-C_8H_{16}NH-\overset{O}{\overset{\|}{C}}-C_8H_{17}$ (derived from N-octylamido-octyl pyrole) | hair hot oil treating lotion | improved conditioning, luster, shine, wet/dry combability & body |
| 15. | 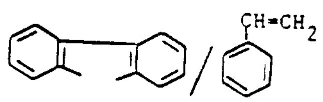 (derived from N-styryl carbazole) | after-shave lotion | easy rubb-in, skin conditioning & lasting effect |
| 16. | 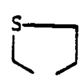 / $-C_{16}H_{33}$ (derived from N-hexadecyl-thiazolidine) | dandruff shampoo & skin medicant | good antibacterial and fungicidal properties |
| 17. | 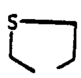 / $-C_{12}H_{25}$ (derived from N-dodecyl-thiazolidine) | dandruff shampoo & skin medicant | good antibacterial and fungicidal properties |
| 18 | $-(CH_2)_4-NH-(CH_2)_4-CH_2-$/ $-C_{12}H_{25}$ (derived from N-dodecyl diazacyclohendecane) | hair shampoo conditioner & setting lotion | improved moisturizing for damaged hair |

* forms a double bond in the heterocyclic ring

Examples of specific formulations for the above uses achieving the benefits noted are presented as follows.

EXAMPLE XXXIX

CREAM RINSE

| Ingredients | Parts by Weight |
|---|---|
| Compound No. 1 in Table III | 2.0 |
| cetyl alcohol | 2.0 |
| emulsifying wax | 2.0 |
| citric acid | to pH 4 |
| deionized water | qs |
| fragrance | qs |
| preservative | qs |

HAIR CONDITIONER

| Ingredients | Parts by Weight |
|---|---|
| Compound No. 2 in Table III | 4.0 |
| PEG-8 Distearate | 2.5 |
| mineral oil | 1.5 |
| lanolin alcohol | 1.0 |
| stearic acid | 1.0 |
| PPG-20 methyl glucose ether | 1.0 |
| hydrolized animal protein | 0.25 |
| citric acid | to pH 4 |
| deionized water | qs |
| preservative | qs |
| fragrance | qs |

BLOW DRY STYLING LOTION

| Ingredients | Parts by Weight |
|---|---|
| Compound No. 3 in Table III | 1.5 |
| ethanol | 3.0 |
| polyquaternium* 11 | 2.0 |
| PEG-10 Castor oil | 0.2 |
| fragrance | 0.2 |
| phosphoric acid | to pH 6 |
| deionized water | qs |

* the quaternized ammonium polymer formed by reacting dimethyl sulfate and a copolymer of vinyl pyrrolidone and dimethylamino methylacrylate

CONDITIONING HAIR SPRAY

| Ingredients | Parts by Weight |
|---|---|
| Compound No. 15 in Table III | 0.6 |
| ethanol | 75.0 |
| ethyl ester of PVM/MA** copolymer | 4.1 |
| 2-amino-2-methyl-1-propanol 99% | 0.1 |
| fragrance | 0.2 |
| propellant | 20.0 |

** vinyl methyl ether/maleic anhydride

CONDITIONING SHAMPOO

| Ingredients | Parts by Weight |
|---|---|
| Compound 11 in Table III | 3.0 |
| N-dodecyl-2-pyrrolidone | 0.6 |
| polyquaternium 11 | 0.5 |
| sodium laureth-4-phosphate | 0.8 |
| ammonium lauryl sulfate | 40.0 |
| silk protein tetrasodium ethylenediamine | 0.25 |
| tetra-acetic acid | 0.2 |
| deionized water | qs |
| colorant | qs |
| fragrance | qs |

MOISTURIZING LOTION

| Ingredients | Parts by Weight |
|---|---|
| Compound No. 9 in Table III | 2.0 |
| mineral oil 70 CTS | 2.0 |
| stearic acid | 3.0 |
| emulsifying wax | 3.0 |
| Dimethicone* 200 CTS | 1.5 |
| Carbomer 934** | 0.15 |
| Oleth-20*** | 1.0 |
| triethanolamine 98% | 1.0 |
| deionized water | qs |
| preservative | qs |
| fragrance | qs |

* a mixture of methylated siloxane polymers end-blocked with trimethyl siloxy units (dimethylpolysiloxane)
** cross-linked polymer of acrylic acid
*** PEG ether of oleyl alcohol

BUBBLE BATH

| Ingredients | Parts by Weight |
|---|---|
| Compound No. 4 in Table III | 3.0 |
| ammonium nonoynol-4-sulfate | 30.0 |
| sodium cocoyl isothionate | 10.0 |
| cocamidopropyl hydroxysultaine | 10.0 |
| cocamide diethanolamide | 6.0 |
| sodium methyl cocyl taurate | 20.0 |
| aloe vera gel | 1.0 |
| coconut oil | 1.0 |
| glycol stearate | 1.0 |
| deionized water | qs |
| preservative | qs |
| colorant | qs |

SHAMPOO FOR OILY HAIR

| Ingredients | Parts by Weight |
|---|---|
| Compound No. 12 in Table III | 3.0 |
| N-dodecyl-2-pyrrolidone | 1.0 |
| tetrasodium ethylenediamine tetra-acetic acid | 0.2 |
| sodium lauryl sulfate | 20.0 |
| alpha-olefin sulfonate | 20.0 |
| polyquaternium 11 | 0.5 |
| deionized water | qs |
| preservative | qs |
| colorant | qs |
| fragrance | qs |
| added inorganic salts as desired for viscosity modification | |

MOUTHWASH

| Ingredients | Parts by Weight |
|---|---|
| Compound No. 6 in Table III | 0.05 |
| alcohol, 190° | 20.00 |
| thymol | 0.03 |
| glycerine | 10.00 |
| flavor | 2.0 |
| distilled water | qs |
| polysorbate 80 | 2.00 |

SYNDET BAR (Superfatted)

| Ingredients | Parts by Weight |
|---|---|
| Compound No. 5 in Table III | 0.5 |
| stearic acid, triple pressed | 32.00 |
| kettle soap | 9.80 |
| sodium cocoyl isethionate | 49.00 |
| sodium methyl cocoyl taurate | 6.90 |
| citric acid, 50% aqueous | 0.60 |
| titanium dioxide | 0.20 |
| fragrance | 1.00 |

WATER RESISTANT EMOLLIENT AFTER SUN LOTION

| Ingredients | Parts by Weight |
|---|---|
| Compound No. 8 in Table III | 3.0 |
| mink Oil, Light Fraction | 11.00 |
| glyceryl stearate, self emulsifying | 1.00 |
| stearic acid | 2.50 |
| mineral oil and lanolin alcohol | 2.00 |
| myristyl myristate | 3.000 |
| mineral oil | 10.00 |
| PVP/Eicosene copolymer | 2.00 |
| triethanolamine | 0.70 |
| sorbitol | 3.00 |
| hydroxyethylcellulose | 0.30 |
| distilled water | qs |
| preservative | qs |
| fragrance | qs |

NON-ALCOHOLIC CONDITIONING MOUSSE

| Ingredients | Parts by Weight |
|---|---|
| Compound No. 6 in Table III | 5.00 |
| PVP K-30 | 2.00 |
| Oleth-2O | 0.50 |
| fragrance | qs |
| deionized water | 77.50 |
| propellant A-46 | 15.00 |

SELF-HEATING AEROSOL SHAVING CREAM

Employed dual dispensing valve for metering oxidant from $H_2O_2$ container and reductant from aerosol can.

| Ingredients | Parts by Weight |
|---|---|
| Compound No. 7 in Table III | 2.00 |
| stripped coconut fatty acid | 1.10 |
| sorbitol | 10.00 |
| stearic acid | 4.20 |
| PEG-40 soritan peroleate | 2.00 |
| triethanolamine | 3.00 |
| potassium hydroxide | 1.00 |
| potassium sulfite | 9.00 |
| fragrance | 0.80 |
| butyrated hydroxy toluene (BHT) | 0.01 |
| butyrated hydroxy anisole (BHA) | 0.01 |
| deionized water | qs |
| propellant | qs |

HAIR MIST CONDITIONER (w/o added preservative)

| Ingredients | Parts by Weight |
|---|---|
| 50/50 Mixture No. 9 & 10 in Table III | 1.00 |
| propylene glycol dicaprylate/dicaprate copolymer | 0.30 |
| oleamidopropyl dimethylamine | 0.50 |
| deionized water | 98.2 |

CATIONIC MOUSSE HAND/BODY LOTION
(Used 85 Parts of the following formula to 15 parts propellant A-46)

| Ingredients | Parts by Weight |
|---|---|
| Compound No. 8 in Table III | 0.50 |
| acetylated polyoxyethylene lanolin | 2.00 |
| ethoxylated lanolin alcohols | 1.00 |
| glyceryl stearate, self-emulsifying | 5.50 |
| cetyl alcohol | 1.50 |
| mineral oil, 70 CTS | 1.50 |
| stearyl alcohol | 1.50 |
| glycerin | 3.00 |
| isopropyl myristate | 4.00 |
| dimethicone, 100 CTS | 2.00 |
| water | qs |
| preservative | qs |
| fragrance | qs |

HOT OIL TREATMENT – (w/o added preservative)

| Ingredients | Parts by Weight |
|---|---|
| 50/50 mixture No. 4 & 14 in Table III | 1.50 |
| oleamidopropyl dimethyl amine | 1.00 |
| polyethylene glycol 6000 distearate | 2.00 |
| hexylene glycol | 4.00 |
| lactic acid, 88% | to pH 4.4 |
| color | qs |
| deionized water | qs |

AFTER SHAVE BALM

| Ingredients | Parts by Weight |
|---|---|
| 50/50 mixture No. 16 & 17 in Table III | 1.00 |
| Carbomer 941 | 0.20 |
| tetrasodium ethylene diamine tetraacetic acid | 0.10 |
| cetearyl alcohol* and polyethylene glycol ether of cetearyl alcohol | 2.50 |
| isopropyl myristate | 1.00 |
| Oleth – 20 | 1.00 |
| methyl gluceth 20 | 2.00 |
| triethanolamine, 98% | 0.20 |
| propylene glycol | 3.00 |
| SDA denatured alcohol | 7.50 |
| PVP/dimethylaminoethyl methacrylate | 7.00 |
| fragrance | 1.00 |
| distilled water | qs |

* 50/50 mixture of cetyl and stearyl alcohols

DANDRUFF SHAMPOO AND SKIN MEDICANT

| Ingredients | Parts by Weight |
|---|---|
| Compound No. 16 in Table III | 4.2% |
| Sodium lauroyl sarcosinate, 30% | 10.0 |
| TEA lauryl sulfate, 40% | 25.0 |
| Magnesium aluminium silicate | 1.0 |
| Hydroxymethyl cellulose, E 4000 | 1.25 |
| Water, perfume, color (D & C Green #5) | qs |

The above examples are representative or preferred embodiments of the present invention; however, it will be understood that other species of instant quaternized lactams can be substituted in the above formulations to provide the benefits indicated. Also, in the preparation of the quaternized lactams described in Examples XXV – XXXVII, other lactam and/or heterocyclic amine reactants described herein can be substituted to provide the corresponding quaternized products which are also included within the scope of this invention. Particularly recommended among these substituted species are the halomethyl – 2 – caprolactam reactants and the amidoalkyl – substituted or hydroxy – substituted heterocyclic amine reactants having from 3 to 8 carbon atoms which also provide useful bactericidal and viscosity enhancing properties.

GROUP D

EXAMPLE XL

To a 1 liter, 4-neck flask equipped with mechanical stirrer, reflux condenser, thermometer, and dropping funnel was added N,N-Dimethyl-N-Octadecyl Amine (150.29 g, 0.505 mole) which was heated with stirring to 70° C. under $N_2$ blanket after which the heating source was removed and N-chloromethyl-2-pyrrolidone (66.8 g, 0.5 mole) was added to the amine dropwise over a period of 25 minutes. An exothermic reaction ensued and was controlled at 100° C. by the rate of addition of N-chloromethyl-2-pyrrolidone. The reaction mixture changed from a liquid to a paste during the addition of N-chloromethyl-2-pyrrolidone and finally to a solid (powder) on completion of the reaction. The yield of N,N-dimethyl-N-octadecyl-N- [(2-pyrrolidonyl)methyl] ammonium chloride, m.p. 110-115° C., was quantitative. The content of the quaternary compound was determined by titration. (Mercuric Acetate method as described by Sidney Siggia, "Quantitative Organic Analysis via Functional Group", 1963, 3d Ed., John Wiley & Sons, pages 552-554).

EXAMPLE XLI

The reaction of Example XL was repeated except that the amine used was N,N-dimethyl-N-hexadecyl amine (2% molar excess with respect to N-chloromethyl-2-pyrrolidone). The product, N,N-dimethyl-N-hexadecyl-N-[(2-pyrrolidonyl) methyl] ammonium chloride, m.p. 98-102° C., was neutralized with acetic acid and recovered in quantitative yield.

EXAMPLE XLII

The reaction of Example XLI was repeated except that the amine used was N,N-dimethyl-N-tetradecyl amine. The corresponding N,N-dimethyl-N-tetradecyl-N-[(2-pyrrolidinyl)methyl] ammonium chloride, m.p. 94-97° C., was recovered in quantitative yield.

EXAMPLE XLIII

The reaction of Example XLI was repeated except that the amine used was N,N-dimethyl-N-dodecyl amine. The corresponding N,N-dimethyl-N-dodecyl-N-[(2-pyrrolidonyl) methyl] ammonium chloride, m.p. 99-103° C., was recovered in quantitative yield.

EXAMPLE XLIV

The reaction of Example XLI was repeated except that the amine used was N,N-dimethyl-N-decyl amine. The corresponding N,N-dimethyl-N-decyl-N-[(2-pyrrolidonyl) methyl] ammonium chloride, m.p. 101-105° C., was recovered in quantitative yield.

EXAMPLE XLV

The reaction of Example XLI was repeated except that the amine used was N,N-dimethyl-N-octyl amine. The corresponding N,N-dimethyl-N-octyl-N-[(2-pyrrolidonyl) methyl] ammonium chloride, m.p. 93-97° C., was recovered in quantitative yield.

EXAMPLE XLVI

The reaction of Example XLI was repeated except that the amine used was N,N-dioctadecyl-N-methyl amine. The corresponding N-methyl-N,N-dioctadecyl-N-[(2-pyrrol idonyl)methyl] ammonium chloride, m.p. 54-58° C., was recovered in quantitative yield.

EXAMPLE XLVII

The reaction of Example XLI was repeated except that the amine used was N,N-didecyl-N-methyl amine. The product, N-methyl-N,N-didecyl-N-[(2-pyrrolidonyl)methyl] ammonium chloride, was obtained as a paste.

Analyses of the products prepared in Examples XL – XLVII are reported in following Table IV.

TABLE IV

ANALYSIS OF TERTIARY AMINE/N-CHLOROMETHYLPYRROLIDONE (CMP) COMPOUNDS

| Tertiary Amine | Equivalents | ANALYSIS * % Quat. | % Free Amine ** |
|---|---|---|---|
| $CH_3(CH_2)_7N(CH_3)_2$ | 1.02 | 97.2 | 0.8 |
| $CH_3(CH_2)_9N(CH_3)_2$ | 1.02 | 97.9 | 2.3 |
| $CH_3(CH_2)_{11}N(CH_3)_2$ | 1.02 | 96.8 | 1.6 |
| $CH_3(CH_2)_{13}N(CH_3)_2$ | 1.02 | 94.5 | 2.5 |
| $CH_3(CH_2)_{15}N(CH_3)_2$ | 1.02 | 98.0 | 1.4 |
| $CH_3(CH_2)_{17}N(CH_3)_2$ | 1.02 | 97.0 | 3.9 |
| $[CH_3(CH_2)_9]_2NCH_3$ | 1.02 | 95.2 | 2.9 |
| $[CH_3(CH_2)_{17}]_2NCH_3$ | 1.02 | 96.1 | 4.5 |

* % quat. and free amine determined by Mercuric Acetate Method

** The free amine titer can be adjusted by the addition of slightly more CMP during preparation.

EXAMPLE XLVIII

The reaction of Example XLI is repeated except that the lactam used is N – bromomethyl – 2 – caprolactam, the N,N – dimethyl – N – hexadecyl – N – [(2 – capronyl)methyl] ammonium bromide is recov –

ered as a solid product.

Other products described herein are prepared by the general procedure outlined above with the substitution of the desired 2 – pyrrolidonyl – , 2 – piperidinonyl – , 2 – capronyl – , 2 – azacycloctanone – , 2 – azacyclononanone – , 2 – azacyclodecanone – or 2 – piperidinone – lactam reactant and/or the substitution of the desired tertiary amine.

EXAMPLE XLIX

Quaternized compounds of the present invention have shown disinfectant properties. The following describes experiments employed to ascertain the disinfectant effect of certain species of the present quaternized compounds against two common bacteria, namely, staphylococcus, aurus and Pseudomonas aeruginosa.

An AOAC use dilution study*was conducted on 8 of the products listed in Table V. More specifically, stainless steel cylinders of 10 mm length and 6 mm inner diameter were immersed in a synthetic broth containing subculture fluid, thioglycollate media USP, for 10 minutes at 20 ° C. after which the contents were dried. The cylinders were then immersed in an aqueous solution of the test product for 10 minutes at 20 ° C. For testing efficacy against staphlococcus aureus, 400 ppm of product in solution was used. For testing efficacy against pseudomonas aeruginosa, 800 ppm of product in solution was employed. The results of these primary tests were recorded and the above procedure was repeated for secondary testing, the results of which were also recorded. Each test was made in 30 replicates and the results reported in Table V show the number of individual cultures in the primary and in the secondary testing where bacterial growth was not arrested.

* "Official Methods of Analysis of the Association of Official Analytical Chemists", 13th Ed., 1980, Chapter 4, para. 4.007-4.011.

TABLE V

Compound Tested

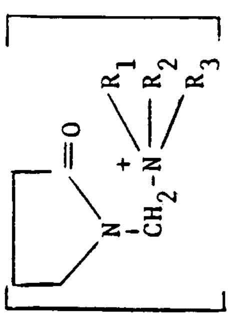

Cl⁻

| $R_1$ | $R_2$ | $R_3$ | Staphlococcus aureus (400 ppm of test compd.) Growth: primary/secondary | Pseudomonas aeruginosa (800 ppm of test compd.) Growth: primary/secondary |
|---|---|---|---|---|
| $CH_3$ | $CH_3$ | $C_8H_{17}$ | 30/30 * | 30/30 * |
| $CH_3$ | $CH_3$ | $C_{10}H_{21}$ | 30/30 * | 0/8 |
| $CH_3$ | $C_{10}H_{21}$ | $C_{10}H_{21}$ | 0/2 | 1/5 |
| $CH_3$ | $CH_3$ | $C_{12}H_{25}$ | 1/3 | 1/1 |
| $CH_3$ | $CH_3$ | $C_{14}H_{29}$ | 1/8 | 2/19 |
| $CH_3$ | $CH_3$ | $C_{16}H_{33}$ | 0/0 ** | 0/1 |
| $CH_3$ | $CH_3$ | $C_{18}H_{37}$ | 1/7 | 8/24 |
| $CH_3$ | $C_{18}H_{37}$ | $C_{18}H_{37}$ | 30/30 * | 30/30 * |

* bacteria growth in all 30 tests
** no bacteria growth in any of the 30 tests

In the 2-pyrrolidonyl series, it appears that the N,N-dimethyl-N-octadecyl-N-[(2-pyrrolidonyl)-methyl] ammonium chloride, the N,N-dimethyl-N-hexadecyl-N-[(2-pyrrolidonyl)methyl] ammonium chloride, the N,N-dimethyl-N-dodecyl-N-[(2-pyrrolidonyl)methyl] ammonium chloride and the N-methyl-N,N-didecyl-N-[(2-pyrrolidonyl) methyl] ammonium chloride products are outstanding in their control of staphylococcus aureus and pseudomonas aeruginosa.

Although all of the present quaternized compounds possess excellent surfactant properties, certain members are particularly recommended for incorporation into specific types of cosmetic formulations. Table VI lists some of such uses for individual quaternized products within the scope of this invention, together with a brief description of the benefits derived from their incorporation.

Generally, the present products are added to the formulations in amounts between about 0.1 and about 5 wt. %, based on active ingredients. The quaternized compounds in Table VI have the general formula:

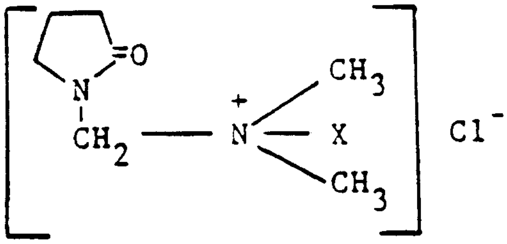

except No. 15 which has the formula:

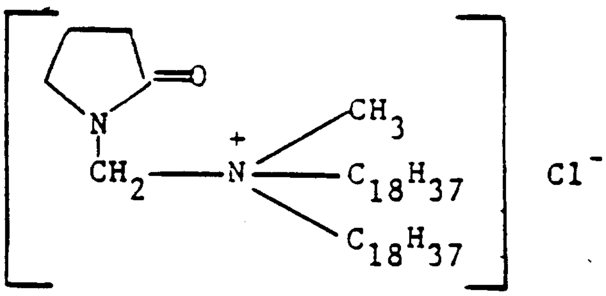

TABLE VI

| No. | Compd where X is | For Formulation In | Benefits Achieved |
|---|---|---|---|
| 1 | n-octadecyl | cream rinse | improved wet/dry combing improved luster & shine |
| 2 | n-octadecyl | hair conditioner | improved wet/dry combing overall conditioning & improved luster & shine |
| 3 | n-hexadecyl | blow-dry styling lotion | protection from heat, improved luster,. shine, body & overall conditioning, preservation and styling |
| 4 | n-octadecyl | moisturizing lotion | facilitates rub-in, smoother feel & high compatability with other components |
| 5 | n-octadecyl | bubble bath | high compatability, smooth after-feel & viscosity enhancement |
| 6 | n-hexadecyl | mouthwash | good germidical properties clean, pleasant taste |
| 7 | n-octadecyl | syndet bar | high compatability & improved feel |
| 8 | n-octadecyl | after-sun lotion | high compatability, smoother feel & facilitates rub-in |
| 9 | n-octadecyl | non-alcoholic mousse | conditioning, improved body, luster & shine, softer feel |
| 10 | n-octadecyl | self-heating aerosol shave cream | high compatability, smoother skin feel, thermal stability & improved spreadability |
| 11 | n-octadecyl | mousse hand & body lotion | improved rub-in and smoother after-feel |

TABLE VI

(continued)

PRESENT COMPOUNDS USED IN MIXTURES FOR INCORPORATION

| | | | |
|---|---|---|---|
| 12 | compd. where X is n-octadecyl + compd where X is n-hexadecyl combined in n-dodecyl-2-pyrrolidone | conditioning shampoo | combined overall conditioning and cleaning, compatability with anionic components, improved wet/dry combing, luster & shine, viscosity enhancement & extra body |
| 13 | compd. where X is n-octadecyl in n-dodecyl-2-pyrrolidone | oily hair shampoo | "greaseless" conditioning, viscosity enhancement & improved wet/dry combability, shine & luster |
| 14 | compd. where X is n-octadecyl + compd where X is n-hexadecyl | hair mist conditioner | improved wet/dry combability, luster & shine, preservation and conditioning |
| | | hot oil treatment | improved conditioning, luster, shine, wet/dry combability, hair body & preservation |
| | | after shave balm | improved rub-in, skin conditioning & preservation and smoother skin feel |
| 15 | n-dioctadecyl | conditoning hair spray | dry compatability, improved luster, shine and manageability. |

Examples of specific formulations for the above uses achieving the benefits noted are presented as follows in Example L.

EXAMPLE L

CREAM RINSE

| Ingredients | Parts by Weight |
|---|---|
| Compound No. 1 in Table VI | 2.0 |
| cetyl alcohol | 2.0 |
| emulsifying wax | 2.0 |
| citric acid | to pH 4 |
| deionized water | qs |
| fragrance | qs |
| preservative | qs |

HAIR CONDITIONER

| Ingredients | Parts by Weight |
|---|---|
| Compound No. 2 in Table VI | 4.0 |
| PEG-8 Distearate | 2.5 |
| mineral oil | 1.5 |
| lanolin alcohol | 1.0 |
| stearic acid | 1.0 |
| PPG-20 methyl glucose ether | 1.0 |
| hydrolized animal protein | 0.25 |
| citric acid | to pH 4 |
| deionized water | qs |
| preservative | qs |
| fragrance | qs |

BLOW DRY STYLING LOTION

| Ingredients | Parts by Weight |
|---|---|
| Compound No. 3 in Table VI | 1.5 |
| ethanol | 3.0 |
| polyquaternium* 11 | 2.0 |
| PEG-10 Castor oil | 0.2 |
| fragrance | 0.2 |
| phosphoric acid | to pH 6 |
| deionized water | qs |

* the quaternized ammonium polymer formed by reacting dimethyl sulfate and a copolymer of vinyl pyrrolidone and dimethylamino methylacrylate

CONDITIONING HAIR SPRAY

| Ingredients | Parts by Weight |
|---|---|
| Compound No. 15 in Table VI | 2.0 |
| ethanol | 75.51 |
| ethyl ester of PVM/MA** copolymer | 4.0 |
| 2-amino-2-methyl-1-propanol 99% | 0.09 |
| fragrance | 0.2 |
| propellant | 20.0 |

** vinyl methyl ether/maleic anhydride

CONDITIONING SHAMPOO

| Ingredients | Parts by Weight |
|---|---|
| Compound mixture of No. 12 in Table VI | |
| X is n-hexadecyl | 1.5 |
| x is n-octadecyl | 1.5 |
| dodecylpyrrolidone | 0.6 |
| polyquaternium 11 | 0.5 |
| sodium laureth-4-phosphate | 0.8 |
| ammonium lauryl sulfate | 40.0 |
| silk protein | 0.25 |
| tetrasodium ethylenediamine tetra-acetic acid | 0.2 |
| deionized water | qs |
| colorant | qs |
| fragrance | qs |

MOISTURIZING LOTION

| Ingredients | Parts by Weight |
|---|---|
| Compound No. 4 in Table VI | 2.0 |
| mineral oil 70 CTS | 2.0 |
| stearic acid | 3.0 |
| emulsifying wax | 3.0 |
| Dimethicone* 200 CTS | 1.5 |
| Carbomer 934** | 0.15 |
| Oleth-20*** | 1.0 |
| triethanolamine 98% | 1.0 |
| deionized water | qs |
| preservative | qs |
| fragrance | qs |

* a mixture of methylated siloxane polymers end-blocked with trimethyl siloxy units (dimethylpolysiloxane)
** cross-linked polymer of acrylic acid
*** PEG ether of oleyl alcohol

BUBBLE BATH

| Ingredients | Parts by Weight |
|---|---|
| Compound No. 5 in Table VI | 3.0 |
| ammonium nonoynol-4-sulfate | 30.0 |
| sodium cocoyl isothionate | 10.0 |
| cocamidopropyl hydroxysultaine | 10.0 |
| cocamide diethanolamide | 6.0 |
| sodium methyl cocyl taurate | 20.0 |
| aloe vera gel | 1.0 |
| coconut oil | 1.0 |
| glycol stearate | 1.0 |
| deionized water | qs |
| preservative | qs |
| colorant | qs |

SHAMPOO FOR OILY HAIR

| Ingredients | Parts by Weight |
|---|---|
| Compound No. 13 in Table VI | 3.0 |
| n-dodecylpyrrolidone | 1.0 |
| tetrasodium ethylenediamine tetra-acetic acid | 0.2 |
| sodium lauryl sulfate | 20.0 |
| alpha-olefin sulfonate | 20.0 |
| polyquaternium 11 | 0.5 |
| deionized water | qs |
| preservative | qs |
| colorant | qs |
| fragrance | qs |
| added inorganic salts as desired for viscosity modification | |

MOUTHWASH

| Ingredients | Parts by Weight |
|---|---|
| Compound No. 6 in Table VI | 0.05 |
| alcohol, 190° | 20.00 |
| thymol | 0.03 |
| glycerine | 10.00 |
| flavor | 2.0 |
| distilled water | qs |
| polysorbate 80 | 2.00 |

SYNDET BAR (Superfatted)

| Ingredients | Parts by Weight |
|---|---|
| Compound No. 7 in Table VI | 0.5 |
| stearic acid, triple pressed | 32.00 |
| kettle soap | 9.80 |
| sodiumcocoyl isethionate | 49.00 |
| sodium methyl cocoyl taurate | 6.90 |
| citric acid, 50% aqueous | 0.60 |
| titanium dioxide | 0.20 |
| fragrance | 1.00 |

WATER RESISTANT EMOLLIENT AFTER SUN LOTION

| Ingredients | Parts by Weight |
|---|---|
| Compound No. 8 in Table VI | 3.0 |
| mink Oil, Light Fraction | 11.00 |
| glyceryl stearate, self emulsifying | 1.00 |
| stearic acid | 2.50 |
| mineral oil and lanolin alcohol | 2.00 |
| myristyl myristate | 3.000 |
| mineral oil | 10.00 |
| PVP/Eicosene copolymer | 2.00 |
| triethanolamine | 0.70 |
| sorbitol | 3.00 |
| hydroxyethylcellulose | 0.30 |
| distilled water | qs |
| preservative | qs |
| fragrance | qs |

NON-ALCOHOLIC CONDITIONING MOUSSE

| Ingredients | Parts by Weight |
|---|---|
| Compound No. 9 in Table VI | 5.00 |
| PVP K-30 | 2.00 |
| Oleth-20 | 0.50 |
| fragrance | qs |
| deionized water | 77.50 |
| propellant A-46 | 15.00 |

SELF-HEATING AEROSOL SHAVING CREAM
(Used dual dispensing valve containing 30 ml stearic acid and 11% hydrogen peroxide)

| Ingredients | Parts by Weight |
|---|---|
| Compound No. 10 in Table VI | 2.00 |
| stripped coconut fatty acid | 1.10 |
| sorbitol | 10.00 |
| stearic acid | 4.20 |
| PEG-40 soritan peroleate | 2.00 |
| triethanolamine | 3.00 |
| potassium hydroxide | 1.00 |
| potassium sulfite | 9.00 |
| fragrance | 0.80 |
| butyrated hydroxy toluene (BHT) | 0.01 |
| butyrated hydroxy anisole (BHA) | 0.01 |
| deionized water | qs |

HAIR MIST CONDITIONER (w/o added preservative)

| Ingredients | Parts by Weight |
|---|---|
| 50/50 Mixture No. 14 in Table VI | 1.00 |
| propylene glycol dicaprylate/dicaprate copolymer | 0.30 |
| oleamidopropyl dimethylamine | 0.50 |
| deionized water | 98.2 |

CATIONIC MOUSSE HAND/BODY LOTION
(Used 85 Parts of the following formula to 15 parts propellant A-46)

| Ingredients | Parts by Weight |
|---|---|
| Compound No. 11 in Table VI | 0.50 |
| acetylated polyoxyethylene lanolin | 2.00 |
| ethoxylated lanolin alcohols | 1.00 |
| glyceryl stearate, self-emulsifying | 5.50 |
| cetyl alcohol | 1.50 |
| mineral oil, 70 CTS | 1.50 |
| stearyl alcohol | 1.50 |
| glycerin | 3.00 |
| isopropyl myristate | 4.00 |
| dimethicone, 100 CTS | 2.00 |
| water | qs |
| preservative | qs |
| fragrance | qs |

HOT OIL TREATMENT – (w/o added preservative)

| Ingredients | Parts by Weight |
|---|---|
| 50/50 mixture No. 14 in Table VI | 1.50 |
| oleamidopropyl dimethyl amine | 1.00 |
| polyethylene glycol 6000 distearate | 2.00 |
| hexylene glycol | 4.00 |
| lactic acid, 88% | to pH 4.4 |
| color | qs |
| deionized water | qs |

AFTER SHAVE BALM

| Ingredients | Parts by Weight |
|---|---|
| 50/50 mixture No. 14 in Table VI | 1.00 |
| Carbomer 941 | 0.20 |
| tetrasodium ethylene diamine tetraacetic acid | 0.10 |
| cetearyl alcohol* and polyethylene glycol ether of cetearyl alcohol | 2.50 |
| isopropyl myristate | 1.00 |
| Oleth-20 | 1.00 |
| methyl gluceth 20 | 2.00 |
| triethanolamine, 98% | 0.20 |
| propylene glycol | 3.00 |
| SDA denatured alcohol | 7.50 |
| PVP/dimethylaminoethyl methacrylate | 7.00 |
| fragrance | 1.00 |
| distilled water | qs |

* 50/50 mixture of cetyl and stearyl alcohols

The above examples are representative or preferred embodiments of the present invention; however, it will be understood that other species of instant quaternized lactams can be substituted in the above formulations to provide the benefits indicated. Also, in the preparation of the quaternized lactams described in Examples XL-XLVIII, other lactam and/or tertiary amine reactants described herein can be substituted to provide the corresponding quaternized products which are also included within the scope of this invention. Particularly recommended among these substituted species are the halomethyl-2-capcolactam reactants and the amidoalkyl-substituted or hydroxy-substituted tertiary amine reactants which also provide useful bactericidal and viscosity enhancing properties.

## Claims

**1.** The quaternized compound having the formula

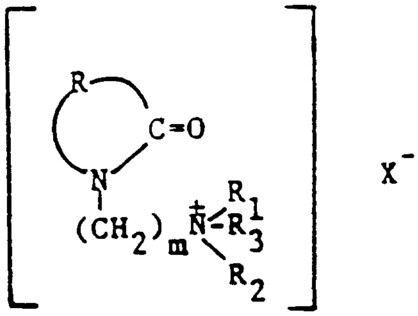

wherein $X^-$ is a chloride, bromide or iodide anion; m is an integer having a value of from 1 to 4; R is $C_3$ to $C_9$ alkylene and is optionally substituted with lower alkyl; and either

(i) $R_1$, $R_2$ and $R_3$ are each independently selected from the group consisting of alkyl, hydroxyalkyl, alkyleneoxyalkyl, alkyleneoxyalkenyl, aryl, alkaryl, aralkyl, aralkenyl, alkyleneamidoalkyl, alkylenecarbamoylalkyl, arylcarbomoylalkyl and arylamidoalkyl radicals each having from 1 to 30 carbon atoms, $R_4-Y-R_7$ or $-(CH_2)_pOR_5$ wherein $R_4$ is alkylene having from 1 to 4 carbon atoms, phenyl or naphthyl optionally substituted with lower alkyl; Y is

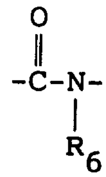

or

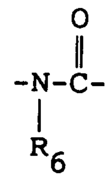

where $R_6$ is hydrogen or lower alkyl; $R_7$ is alkyl of from 1 to 30 carbon atoms, p is an integer having a value from 1 to 4, and $R_5$ is alkyl or alkenyl having from 1 to 30 carbon atoms, and at least one of $R_1$, $R_2$, $R_3$, $R_5$ and $R_7$ having from 8 to 30 carbon atoms, or

(ii) $R_2$ and $R_3$ together with the quaternary nitrogen atom form a 5 to 14 membered heterocyclic ring having from 1 to 2 heteroatoms of the group oxygen, sulfur and nitrogen, while $R_1$ is a said radical having from 1 to 30 carbon atoms or represents a double bond in the quaternized heterocyclic ring formed by $R_2$ and $R_3$, but excluding the compound

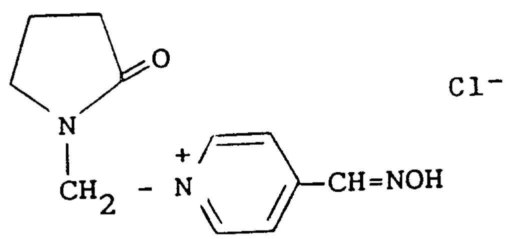

**2.** The quaternized compound of Claim 1 wherein m has a value of 1; R taken together with $-CO$ and $-N-$ forms a pyrrolidonyl or a caprolactam ring and $X^-$ is a chloride anion.

**3.** The quaternized compound of Claim 2 wherein $R_1$, $R_2$ and $R_3$ are alkyl.

**4.** The quaternized compound of Claim 3 wherein at least one of $R_1$, $R_2$ and $R_3$ is alkyl having from 8 to 22 carbon atoms and another of $R_1$, $R_2$ and $R_3$ is methyl.

**5.** The quaternized compound of Claim 4 which is the N,N-dimethyl-N-octadecyl-N-[(2-pyrrolidonyl)methyl] ammonium salt,
the N-methyl-N,N-dioctadecyl-N-[(2-pyrrolidonyl)methyl] ammonium salt,
the N,N-dimethyl-N-hexadecyl-N-[(2-pyrrolidonyl)methyl] ammonium salt,
the N,N-dimethyl-N-decyl-N-[(2-pyrrolidonyl)methyl] ammonium salt,
the N-methyl-N,N-didecyl-N-[(2-pyrrolidonyl)methyl] ammonium salt, or
the N,N-dimethyl-N-dodecyl-N-[(2-pyrrolidonyl)methyl] ammonium salt.

**6.** The quaternized compound of Claim 1 having the formula

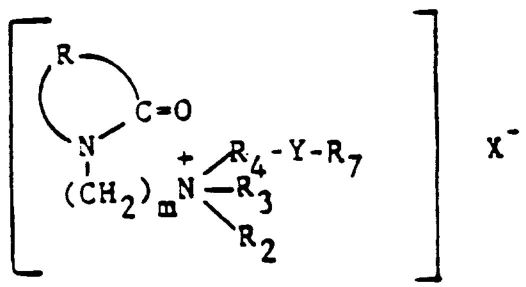

wherein m is an integer having a value of from 1 to 4; R is alkylene having from 3 to 8 carbon atoms and is optionally substituted with $C_1$ to $C_4$ alkyl; $R_4$ is alkylene having from 1 to 4 carbon atoms, phenyl or naphthyl optionally substituted with lower alkyl; Y is

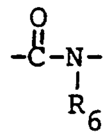 or 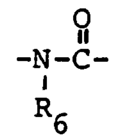

where $R_6$ is hydrogen or lower alkyl; $R_7$ is alkyl of from 1 to 30 carbon atoms; $R_2$ and $R_3$ are each independently $-R_4-Y-R_7$ or a radical selected from alkyl, alkyleneoxyalkyl, alkylhydroxy, aryl, aralkyl, aralkenyl, alkaryl and alkylamidoalkyl radicals having from 1 to 30 carbon atoms and at least one of $R_7$, $R_2$ and $R_3$ being a radical having at least 8 carbon atoms and $X^-$ is a chloride, bromide or iodide anion.

**7.** The quaternized compound of Claim 6 wherein m has a value of 1; R taken together with $-CO$ and $-N-$ forms a 5 or 6 membered ring; $X^-$ is a chloride anion and one of $R_7$, $R_2$ and $R_3$ is methyl.

**8.** The quaternized compound of Claim 7 wherein at least one of $R_7$, $R_2$ and $R_3$ is alkyl having from 8 to 22 carbon atoms and another of $R_7$, $R_2$ and $R_3$ is methyl.

**9.** The quaternized compound of Claim 8 which is N,N-dimethyl-N-[(2-pyrrolidonyl)methyl]-N-(3-octadecanamidopropyl) ammonium chloride,
N,N-dimethyl-N-[(2-pyrrolidonyl)methyl]-N-(3-docosanamidopropyl) ammonium chloride,
N,N-dimethyl-N-[(2-azacycloheptanoyl)methyl]-N-(3-octadecanamidopropyl) ammonium chloride,
N,N-dimethyl-N-[(2-pyrrolidonyl)methyl]-N-(3-dodecanamidopropyl) ammonium chloride,
N,N-dimethyl-N-[(2-pyrrolidonyl)methyl]-N-(2-octadecanamidoethyl) ammonium chloride, or
the N,N-dimethyl-N-[(2-piperidonyl)methyl]-N-(2-octadecanamidoethyl) ammonium chloride.

**10.** The quaternized compound of Claim 1 having the formula

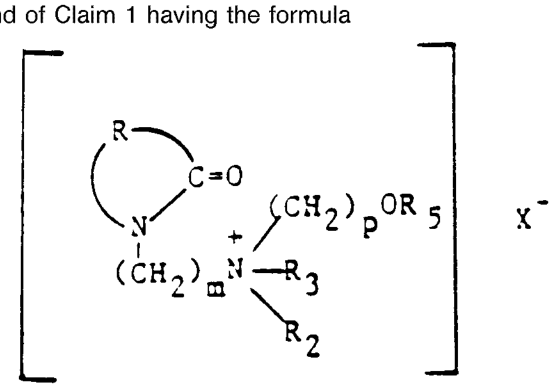

wherein $X^-$ is a chloride, bromide or iodide anion; m and p are each integers having a value of from 1 to 4; R is $C_3$ to $C_9$ alkylene in which alkylene may be substituted with lower alkyl; $R_5$ is alkyl or alkenyl

having from 1 to 30 carbon atoms and $R_2$ and $R_3$ are each independently $-(CH_2)_pOR_1$ or a radical selected from alkyl, hydroxyalkyl, aryl, alkaryl, aralkyl, aralkenyl, and alkylamidoalkyl radicals having from 1 to 30 carbon atoms and at least one of $R_5$, $R_2$ and $R_3$ is a radical containing from 8 to 30 carbon atoms.

**11.** The quaternized compound of Claim 10 wherein m has a value of 1; R taken together with $-CO$ and $-N-$ forms a pyrrolidonyl or a azacycloheptanonyl ring; $X^-$ is a chloride anion and $R_2$ and $R_3$ are alkyl.

**12.** The quaternized compound of Claim 11 wherein at least one of $R_5$, $R_2$ and $R_3$ is alkyl having from 8 to 22 carbon atoms and another of $R_5$, $R_2$ and $R_3$ is methyl.

**13.** The quaternized compound of Claim 12 which is the N,N-dimethyl-N-[(2-pyrrolidonyl)methyl]-N-[3-(octadecyloxy)propyl] ammonium salt,
the N,N-dimethyl-N-[(2-pyrrolidonyl)methyl]-N-[3-(hexadecyloxy)propyl] ammonium salt,
the N,N-dimethyl-N-[(2-azacycloheptanoyl)methyl]-N-[3-(octadecyloxy)propyl] ammonium salt,
the N,N-dimethyl-N-[(2-piperidonyl)methyl]-N-[3-(dodecyloxy)propyl] ammonium salt,
the N,N-diethyl-N-[(2-pyrrolidonyl)methyl]-N-[3-(octadecyloxy)propyl] ammonium salt, or
the N-methyl-N-[(2-pyrrolidonyl)methyl]N,N-bis[3-(octadecyloxy)propyl] ammonium salt.

**14.** The quaternized compound of Claim 1 having the formula

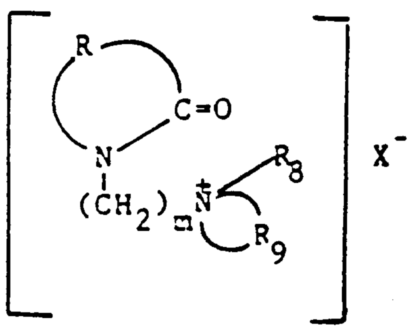

wherein $X^-$ is a chloride, bromide or iodide anion; m is an integer having a value of from 1 to 4; R is alkylene having from 3 to 8 carbon atoms and is optionally substituted with $C_1$ to $C_4$ alkyl; $R_8$ forms a double bond in the heterocyclic ring with the quaternized nitrogen or is selected from the group consisting of alkyl, hydroxyalkyl, alkyleneoxyalkyl, aryl, alkaryl, aralkyl, aralkenyl and alkylamidoalkyl and $R_9$, together with the quaternary nitrogen atom forms a 5 to 14 membered heterocyclic structure containing up to 2 hetero atoms selected from the group of nitrogen, oxygen and sulfur.

**15.** The compound of Claim 14 wherein R is alkylene having up to 4 carbon atoms and is optionally substituted with lower alkyl.

**16.** The compound of Claim 14 wherein $R_9$ forms a saturated heterocyclic ring with the quaternized nitrogen and wherein $R_8$ contains from 8 to 22 carbon atoms.

**17.** The compound of Claim 16 wherein $R_9$ forms a pyrrolidinyl ring, a piperidinyl ring, or a morpholinyl ring.

**18.** The compound of Claim 14 wherein the heterocyclic ring formed between $R_9$ and the quaternized nitrogen atom contains a double bond and $R_8$ represents a double bond in said ring or is a group bonded to the quaternized nitrogen atom and contains from 8 to 22 carbon atoms.

**19.** The compound of Claim 18 wherein $R_9$ forms an imidazole ring and $R_8$ contains from 8 to 22 carbon atoms.

**20.** The compound of Claim 18 wherein $R_9$ forms a pyridinyl ring or an oxazolidinyl ring and $R_8$ represents a double bond within said ring.

**21.** The compound of Claim 14 wherein $R_9$ together with the quaternized nitrogen atom forms a 5 to 10 membered heteromonocyclic ring, a 9 to 10 membered heterobicyclic ring, or a 13 to 14 membered heterotricyclic ring.

**22.** The process for preparing the quaternized compound of Claim 1 which comprises reacting a lactam having the formula

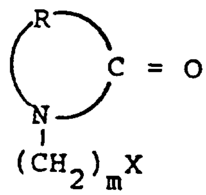

with a tertiary amine coreactant having the formula

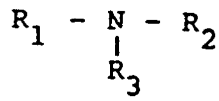

wherein R, m, $R_1$, $R_2$ and $R_3$ are as defined in Claim 1 and X is selected from the group of chloro, bromo and iodo; at a temperature of between about 25° and about 150° C. under a pressure of from 0 to 0.34 MPa (50 psig).

**23.** The process of Claim 22 wherein lactam is N-chloromethyl-2-pyrrolidone.

**24.** The process of Claim 23 wherein the tertiary amine has the formula

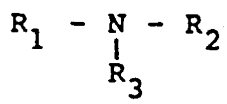

wherein $R_1$ is methyl, $R_2$ is methyl or a radical containing 8 to 22 carbon atoms and $R_3$ is a radical containing 8 to 22 carbon atoms.

**25.** The process of Claim 23 wherein the reaction is effected under atmospheric pressure at a temperature between about 60° and about 100° C.

**26.** The process of Claim 22 wherein the amine coreactant is selected from the group of

A. 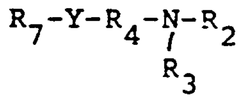 ,

B. 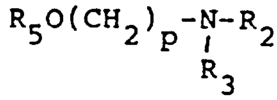 ,

C. 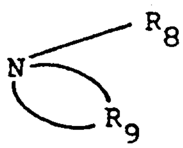 , and

D. 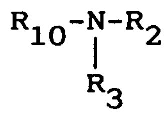

wherein $R_2$ and $R_3$ are as defined in Claim 1; Y, $R_4$ and $R_7$ are as defined in Claim 6; $R_5$ and p are as defined in Claim 10; $R_8$ and $R_9$ are as defined in Claim 14 and $R_{10}$ is alkyl having from 8 to 30 carbon atoms.

**27.** Use of a compound according to any one of Claims 1 to 21 as a hair conditioner in a shampoo formulation.

**28.** Use according to Claim 27 wherein the shampoo formulation contains N-methyl-N,N-dioctadecyl-N-[(Z-pyrrolidonyl)methyl]ammonium salt as a preservative.

**29.** Use of a compound according to any one of Claims 1 to 21 as from 0.5 wt% to 5 wt% of a shampoo formulation which contains a surfactant which is an alkali metal lauryl sulfate, an alkali metal lauryl sulfate ether, ammonium lauryl sulfate or an $\alpha$-olefin sulfonate.

**30.** Use of a compound according to any one of Claims 1 to 21 as a bactericide in a liquid subject to bacterial degradation.

**31.** Use of the compound of Claim 14, wherein the quaternized heterocyclic ring contains a sulfur atom, as a bactericide in a liquid subject to bacterial degradation.

**32.** Use of a compound according to any one of Claims 1 to 21 to increase viscosity of a liquid.

**33.** Use of a compound according to any one of Claims 1 to 21 to increase viscosity of a hair or skin treating formulation.

**34.** Use of a compound according to any one of claims 1 to 21 as a dye leveler in a dye bath.

**35.** Use of a compound according to any one of Claims 1 to 21 as a textile softening and antistatic agent in a laundry detergent formulation.

**36.** Use of a compound according to any one of Claims 1 to 21 as a conditioner in a cosmetic formulation.

**37.** A cosmetic formulation containing from 0.05 to 8 wt% of a compound according to any one of claims 1 to 21.

**38.** A textile treating formulation containing from 0.05 to 8 wt% of a compound according to any one of Claims 1 to 21.

## Patentansprüche

**1.** Quaternisierte Verbindung der Formel

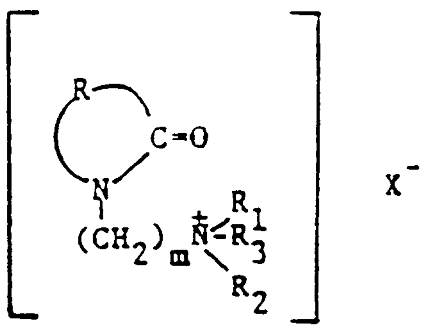

worin $X^-$ ein Chlorid-, Bromid- oder Jodidanion ist; m eine ganze Zahl mit einem Wert von 1 bis 4 ist; R $C_3-C_9$-Alkylen ist und wahlweise mit Niederalkyl substituiert ist; und entweder

(i) $R_1$, $R_2$ und $R_3$ jeweils unabhängig aus der Gruppe ausgewählt sind, die aus Alkyl-, Hydroxyalkyl-, Alkylenoxyalkyl-, Alkylenoxyalkenyl, Aryl-, Alkaryl-, Aralkyl-, Aralkenyl-, Alkylenamidoalkyl-, Alkylencarbamoylalkyl-, Arylcarbamoylalkyl- und Arylamidoalkylresten besteht, die jeweils von 1 bis 30 C-Atomen aufweisen, $R_4-Y-R_7$ oder $-(CH_2)_pOR_5$, worin $R_4$ Alkylen mit von 1 bis 4 C-Atomen, Phenyl oder Naphthyl ist, wahlweise mit Niederalkyl substituiert; Y

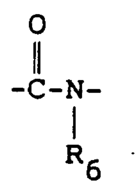

oder

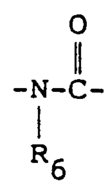

ist, worin $R_6$ Wasserstoff oder Niederalkyl ist; $R_7$ ein Alkyl mit von 1 bis 30 C-Atomen ist, p eine ganze Zahl mit einem Wert von 1 bis 4 ist und $R_5$ Alkyl oder Alkenyl mit von 1 bis 30 C-Atomen ist und zumindest eines aus $R_1$, $R_2$, $R_3$, $R_5$ und $R_7$ von 8 bis 30 C-Atomen aufweist, oder

(ii) $R_2$ und $R_3$ gemeinsam mit dem quaternären Stickstoffatom einen 5- bis 14-gliedrigen heterozyklischen Ring mit von 1 bis 2 Heteroatomen der Gruppe Sauerstoff, Schwefel und Stickstoff bilden, während $R_1$ ein genannter Rest mit von 1 bis 30 C-Atomen ist oder eine Doppelbindung im durch $R_2$ und $R_3$ gebildeten quaternisierten heterozyklischen Ring darstellt, aber mit Ausnahme der Verbindung

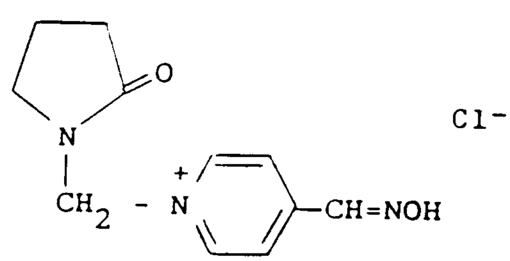

2. Quaternisierte Verbindung nach Anspruch 1, worin m einen Wert von 1 hat; R gemeisnam mit −CO und −N− einen Pyrrolidonyl− oder einen Caprolactamring bildet und $X^-$ ein Chloridanion ist.

3. Quaternisierte Verbindung nach Anspruch 2, worin $R_1$, $R_2$ und $R_3$ Alkyl sind.

4. Quaternisierte Verbindung nach Anspruch 3, worin zumindest eines aus $R_1$, $R_2$ und $R_3$ Alkyl mit von 8 bis 22 C−Atomen ist und ein anderes aus $R_1$, $R_2$ und $R_3$ Methyl ist.

5. Quaternisierte Verbindung nach Anspruch 4, die das N,N−Dimethyl−N−octadecyl−N−[(2−pyrrolidonyl)methyl]ammoniumsalz,
das N−Methyl−N,N−dioctadecyl−N−[(2−pyrrolidonyl)methyl]−Ammoniumsalz,
das N−N−Dimethyl−N−hexadecyl−N−[(2−pyrrolidonyl)methyl]−Ammoniumsalz,
das N,N−Dimethyl−N−decyl−N−[(2−pyrrolidonyl)methyl]−Ammoniumsalz,
das N−Methyl−N,N−didecyl−N−[(2−pyrrolidonyl)methyl]−Ammoniumsalz oder
das N,N−Dimethyl−N−dodecyl−N−[(2−pyrrolidonyl)methyl]−Ammoniumsalz ist.

6. Quaternisierte Verbindung nach Anspruch 1 der Formel

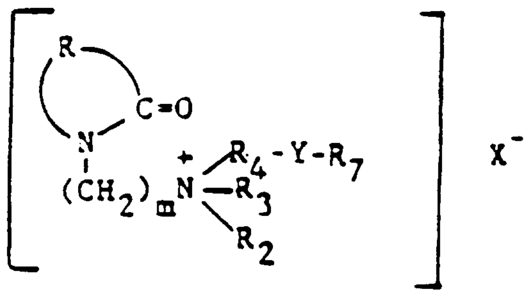

worin m eine ganze Zahl mit einem Wert von 1 bis 4 ist; R Alkylen mit 3 bis 8 C−Atomen ist und wahlweise mit $C_1-C_4$−Alkyl substituiert ist; $R_4$ Alkylen mit von 1 bis 4 C−Atomen, Phenyl oder Naphthyl ist, das wahlweise mit Niederalkyl substituiert ist; Y

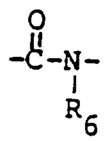

oder

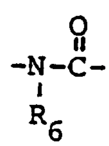

ist, worin $R_6$ Wasserstoff oder Niederalkyl ist; $R_7$ Alkyl mit von 1 bis 30 C−Atomen ist; $R_2$ und $R_3$ jeweils unabhängig $-R_4-Y-R_7$ oder ein Rest ausgewählt aus Alkyl−, Alkylenoxyalkyl−, Alkylhydroxy−, Aryl−, Aralkyl−, Aralkenyl−, Alkaryl− und Alkylamidoalkylresten mit von 1 bis 30 C−Atomen sind, und wobei zumindest eines aus $R_7$, $R_2$ und $R_3$ ein Rest mit zumindest 8 C−Atomen ist und $X^-$ ein Chlorid−, Bromid− oder Jodidanion ist.

7. Quaternisierte Verbindung nach Anspruch 5, worin m einen Wert von 1 hat; R gemeinsam mit −CO und −N− einen 5− oder 6−gliedrigen Ring bildet; $X^-$ ein Chloridanion ist und eines aus $R_7$, $R_2$ und $R_3$ Methyl ist.

8. Quaternisierte Verbindung nach Anspruch 7, worin zumindest eines aus $R_7$, $R_2$ und $R_3$ Alkyl mit von 8 bis 22 C−Atomen ist und ein anderes aus $R_7$, $R_2$ und $R_3$ Methyl ist.

9. Quaternisierte Verbindung nach Anspruch 8, die N,N−Dimethyl−N−[(2−pyrrolidonyl)methyl]−N−(3−octadecanamidopropyl)−Ammoniumchlorid,
N,N−Dimethyl−N−[(2−pyrrolidonyl)methyl]−N−(3−docosanamidopropyl)−Ammoniumchlorid,
N,N−Dimethyl−N−[(2−azacycloheptanoyl)methyl]−N−(3−octadecanamidopropyl)−Ammoniumchlorid,
N,N−Dimethyl−N−[(2−pyrrolidonyl)methyl]−N−(3−dodecanamidopropyl)−Ammoniumchlorid,
N,N−Dimethyl−N−[(2−pyrrolidonyl)methyl]−N−(2−octadecanamidoäthyl)−Ammoniumchlorid oder

das N,N-Dimethyl-N-[(2-piperidonyl)methyl]-N-(2-octadecanamidoäthyl)-Ammoniumchlorid ist.

**10.** Quaternisierte Verbindung nach Anspruch 1 der Formel

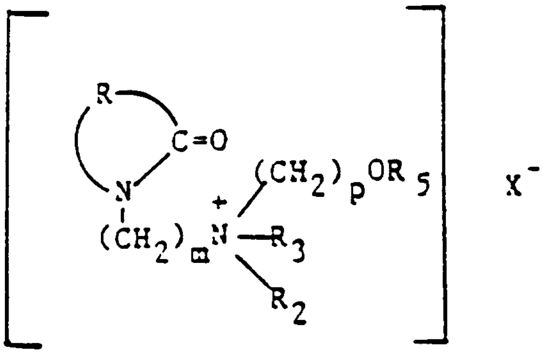

worin $X^-$ ein Chlorid-, Bromid- oder Jodidanion ist; m und p jeweils ganze Zahlen mit einem Wert von 1 bis 4 sind; R $C_3-C_9$-Alkylen ist, worin Alkylen mit Niederalkyl substituiert sein kann; $R_5$ Alkyl oder Alkenyl mit von 1 bis 30 C-Atomen ist und $R_2$ und $R_3$ jeweils unabhängig $-(CH_2)_pOR_1$ oder ein Rest ausgewählt aus Alkyl-, Hydroxyalkyl-, Aryl-, Alkaryl-, Aralkyl-, Aralkenyl- und Alkylamidoalkylresten mit von 1 bis 30 C-Atomen sind und zumindest eines aus $R_5$, $R_2$ und $R_3$ ein Rest ist, der von 8 bis 30 C-Atome enhält.

**11.** Quaternisierte Verbindung nach Anspruch 10, worin m einen Wert von 1 hat; R gemeinsam mit -CO und -N- einen Pyrrolidonyl- oder einen Azacycloheptanonylring bildet; $X^-$ ein Chloridanion ist und $R_2$ und $R_3$ Alkyl sind.

**12.** Quaternisierte Verbindung nach Anspruch 11, worin zumindest eines aus $R_5$, $R_2$ und $R_3$ Alkyl mit von 8 bis 22 C-Atomen ist und ein anderes aus $R_5$, $R_2$ und $R_3$ Methyl ist.

**13.** Quaternisierte Verbindung nach Anspruch 12, die das N,N-Dimethyl-N-[(2-pyrrolidonyl)methyl)-N-[3-(octadecyloxy)propyl]-Ammoniumsalz,
das N,N-Dimethyl-N-[(2-pyrrolidonyl)methyl]-N-[3-hexadecyloxy)propyl]-Ammoniumsalz,
das N,N-Dimethyl-N-[(2-azacycloheptanoyl)methyl]-N-[3-(octadecyloxy)propyl]-Ammoniumsalz,
das N,N-Dimethyl-N-[(2-piperidonyl)methyl]-N-[3-(dodecyloxy)propyl]-Ammoniumsalz,
das N,N-Diäthyl-N-[(2-pyrrolidonyl)methyl]-N-[3-(octadecyloxy)propyl]-Ammoniumsalz oder
das N-Methyl-N-[(2-pyrrolidonyl)methyl]-N,N-bis[3-(octadecyloxy)propyl]-Ammoniumsalz ist.

**14.** Quaternisierte Verbindung nach Anspruch 1 der Formel

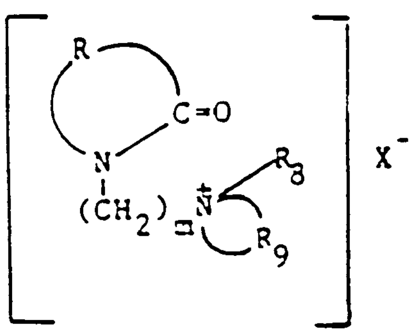

worin $X^-$ ein Chlorid-, Bromid- oder Jodidanion ist; m eine ganze Zahl mit einem Wert von 1 bis 4 ist; R ein Alkylen mit von 3 bis 8 C-Atomen ist und wahlweise mit $C_1-C_4$-Alkyl substituiert ist; $R_8$ eine Doppelbindung im heterozyklischen Ring mit dem quaternisierten Stickstoff bildet oder aus der

Gruppe ausgewählt ist, die aus Alkyl, Hydroxyalkyl, Alkylenoxyalkyl, Aryl, Alkaryl, Aralkyl, Aralkenyl und Alkylamidoalkyl besteht, und $R_9$ gemeinsam mit dem quaternären Stickstoffatom eine 5– bis 14–gliedrige heterozyklische Struktur bildet, die bis zu 2 Heteroatome enthält, die aus der Gruppe von Stickstoff, Sauerstoff und Schwefel ausgewählt sind.

**15.** Verbindung nach Anspruch 14, worin R Alkylen mit bis zu 4 C–Atomen ist und wahlweise mit Niederalkyl substituiert ist.

**16.** Verbindung nach Anspruch 14, worin $R_9$ einen gesättigten heterozyklischen Ring mit dem quaternisierten Stickstoff bildet und worin $R_8$ von 8 bis 22 C–Atome enthält.

**17.** Verbindung nach Anspruch 16, worin $R_9$ einen Pyrrolidinylring, einen Piperidinylring oder einen Morpholinylring bildet.

**18.** Verbindung nach Anspruch 14, worin der zwischen $R_9$ und dem quaternisierten Stickstoffatom gebildete heterozyklische Ring eine Doppelbindung enthält und $R_8$ eine Doppelbindung im genannten Ring darstellt oder eine an das quaternisierte Stickstoffatom gebundene Gruppe ist und von 8 bis 22 C–Atome enthält.

**19.** Verbindung nach Anspruch 18, worin $R_9$ einen Imidazolring bildet und $R_8$ von 8 bis 22 C–Atome enthält.

**20.** Verbindung nach Anspruch 18, worin $R_9$ einen Pyridinylring oder einen Oxazolidinylring bildet und $R_8$ eine Doppelbindung innerhalb des genannten Rings darstellt.

**21.** Verbindung nach Anspruch 14, worin $R_9$ gemeinsam mit dem quaternisierten Stickstoffatom einen 5– bis 10–gliedrigen heteromonozyklischen Ring, einen 9– bis 10–gliedrigen heterobizyklischen Ring oder einen 13– bis 14–gliedrigen heterotrizyklischen Ring bildet.

**22.** Verfahren zur Herstellung einer quaternisierten Verbindung nach Anspruch 1, welches das Umsetzen eines Laktams der Formel

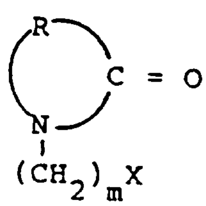

mit einem tertiären Aminkoreaktanden der Formel

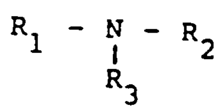

unfaßt, worin R, m, $R_1$, $R_2$ und $R_3$ die in Anspruch 1 definierte Bedeutung haben und X aus der Gruppe aus Chlor, Brom und Jod ausgewählt ist; bei einer Temperatur von zwischen etwa 25° und etwa 150°C unter einem Druck von 0 bis 0,34 MPa (50 psig).

**23.** Verfahren nach Anspruch 22, worin das Laktam N–Chlormethyl–2–pyrrolidon ist.

**24.** Verfahren nach Anspruch 23, worin das tertiäre Amin die Formel

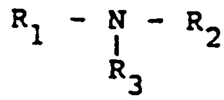

hat, worin $R_1$ Methyl ist, $R_2$ Methyl oder ein Rest ist, der 8 bis 22 C – Atome enthält, und $R_3$ ein Rest ist, der 8 bis 22 C – Atome enthält.

**25.** Verfahren nach Anspruch 23, worin die Umsetzung unter atmosphärischem Druck bei einer Temperatur zwischen etwa 60 ° und etwa 100 ° C bewirkt wird.

**26.** Verfahren nach Anspruch 22, worin der Amincoreaktand aus der Gruppe aus

A. 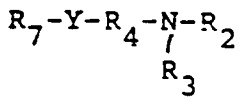

B. 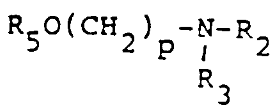 ,

C. 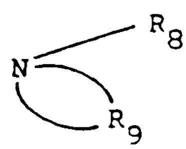 , und

D. 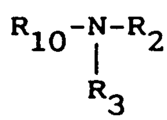

ausgewählt ist, worin $R_2$ und $R_3$ die in Anspruch 1 definierte Bedeutung haben; Y, $R_4$ und $R_7$ die in Anspruch 6 definierte Bedeutung haben; $R_5$ und p die in Anspruch 10 definierte Bedeutung haben; $R_8$ und $R_9$ die in Anspruch 14 definierte Bedeutung haben und $R_{10}$ Alkyl mit von 8 bis 30 C – Atomen ist.

**27.** Verwendung einer Verbindung nach einem der Ansprüche 1 bis 21 als Haarconditioner in einer Shampooformulierung.

**28.** Verwendung nach Anspruch 27, worin die Shampooformulierung N – Methyl – N,N – dioctadecyl – N – [ – (2 – pyrrolidonyl)methyl] – Ammoniumsalz als ein Konservierungsmittel enthält.

**29.** Verwendung einer Verbindung nach einem der Ansprüche 1 bis 21 als von 0,5 Gew. – % bis 5 Gew. – % einer Shampooformulierung, die ein Tensid enthält, das ein Alkalimetalllaurylsulfat, ein Alkalimetall – laurylsulfatäther, Ammoniumlaurylsulfat oder ein $\alpha$ – Olefinsulfonat ist.

**30.** Verwendung einer Verbindung nach einem der Ansprüche 1 bis 21 als ein Bakterizid in einer Flüssigkeit, die bakteriellem Abbau unterworfen ist.

**31.** Verwendung einer Verbindung nach Anspruch 14, worin der quaternisierte heterozyklische Ring ein Schwefelatom enthält, als ein Bakterizid in einer Flüssigkeit, die bakteriellem Abbau unterworfen ist.

**32.** Verwendung einer Verbindung nach einem der Ansprüche 1 bis 21, um die Viskosität einer Flüssigkeit zu erhöhen.

**33.** Verwendung einer Verbindung nach einem der Ansprüche 1 bis 21, um die Viskosität einer Haar- oder Hautbehandlungsformulierung zu erhöhen.

**34.** Verwendung einer Verbindung nach einem der Ansprüche 1 bis 21 als ein Farbausgleichmittel in einem Färbebad.

**35.** Verwendung einer Verbindung nach einem der Ansprüche 1 bis 21 als ein Textilweichmacher und Antistatikmittel in einer Waschmittelformulierung.

**36.** Verwendung einer Verbindung nach einem der Ansprüche 1 bis 21 als ein Conditioner in einer Kosmetikformulierung.

**37.** Kosmetikformulierung, die von 0,05 bis 8 Gew.-% einer Verbindung nach einem der Ansprüche 1 bis 21 enthält.

**38.** Textilbehandlungsformulierung, die von 0,05 bis 8 Gew.-% einer Verbindung nach einem der Ansprüche 1 bis 21 enthält.

**Revendications**

**1.** Composé quaternisé ayant pour formule

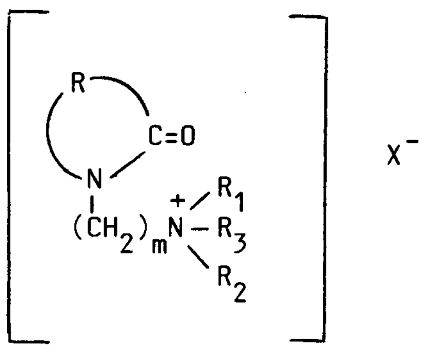

où $X^-$ est un anion de chlorure, bromure ou iodure ; m est un nombre entier ayant une valeur comprise entre 1 et 4 ; R est alkylène $C_3$ à $C_9$ et est facultativement substitué par un alkyle inférieur ; et soit

(i) chacun de $R_1$ , $R_2$ et $R_3$ est indépendamment choisi dans le groupe consistant en radicaux alkyle, hydroxyalkyle, alkylèneoxyalkyle, alkylèneoxyalkényle, aryle, alcaryle, aralkyle, aralkényle, alkylèneamidoalkyle, alkylènecarbamoylalkyle, arylcarbamoylalkyle et arylamidoalkyle ayant chacun de 1 à 30 atomes de carbone, $R_4-Y-R_7$ ou $-(CH_2)_pOR_5$ où $R_4$ est alkylène ayant de 1 à 4 atomes de carbone, phényle ou naphtyle facultativement substitué par alkyle inférieur ; Y est

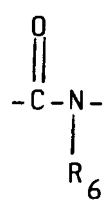 ou 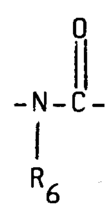

où $R_6$ est hydrogène ou alkyle inférieur ; $R_7$ est alkyle de 1 à 30 atomes de carbone p est un nombre entier ayant une valeur comprise entre 1 et 4 et $R_5$ est alkyle ou alkényle ayant de 1 à 30 atomes de carbone et au moins l'un de $R_1$, $R_2$, $R_3$, $R_5$ et $R_7$ à de 8 à 30 atomes de carbone, ou

(ii) $R_2$ et $R_3$ avec l'atome d'azote quaternaire forment un noyau hydrocyclique de 5 à 14 membres ayant 1 à 2 hétéroatomes du groupe oxygàne, soufre et azote, tandis que $R_1$ est un radical ayant de

1 à 30 atomes de carbone ou représente une double liaison dans le noyau hétérocyclique quaternisé formé par $R_2$ et $R_3$, mais à l'exclusion du composé

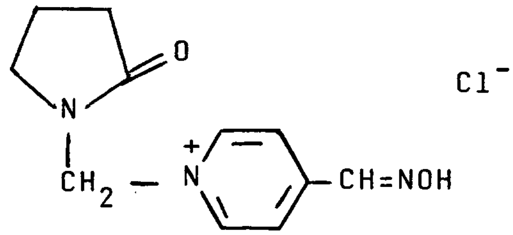

2. Composé quaternisé de la revendication 1, où m a une valeur de 1; R ,pris avec −CO et −N−,forme un noyau de pyrrolidonyle ou de caprolactame et $X^-$ est un anion de chlorure.

3. Composé quaternisé de la revendication 2, où $R_1$, $R_2$ et $R_3$ sont alkyle.

4. Composé quaternisé de la revendication 3, où au moins l'un de $R_1$, $R_2$ et $R_3$ est alkyle ayant 8 à 22 atomes de carbone et un autre de $R_1$, $R_2$ et $R_3$ est méthyle.

5. Composé quaternisé de la revendication 4, qui est le sel de N,N−diméthyl−N−octadécyl−N−[(2−pyrrolidonyl)−méthyl]ammonium,
le sel de N−méthyl−N,N−dioctadécyl−N−[(2−pyrrolidonyl)−méthyl]ammonium,
le sel de N,N−diméthyl−N−hexadécyl−N−[(2−pyrrolidonyl)−méthyl]ammonium,
le sel de N,N−diméthyl−N−décyl−N−[(2−pyrrolidonyl)méthyl]ammonium,
le sel de N−méthyl−N,N−dicétyl−N−[(2−pyrrolidonyl)−méthyl]ammonium, ou
le sel de N,N−diméthyl−N−dodécyl−N−[(2−pyrrolidonyl)−méthyl]ammonium.

6. Composé quaternisé de la revendication 1 ayant pour formule

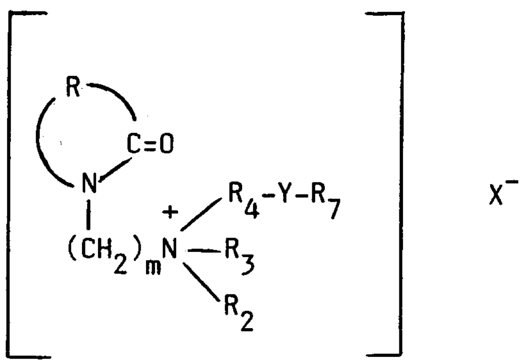

où m est un nombre entier ayant une valeur comprise entre 1 et 4 ; R est alkylène ayant 3 à 8 atomes de carbone et est facultativement substitué par un alkyle $C_1$ à $C_4$ ; $R_4$ est alkylène ayant 1 à 4 atomes de carbone, phényle ou naphtyle facultativement substitué par alkyle inférieur ; Y est

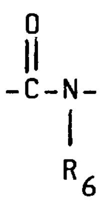

ou

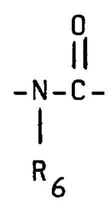

où $R_6$ est hydrogène ou alkyle inférieur ; $R_7$ est alkyle de 1 à 30 atomes de carbone ; chacun de $R_2$ et $R_3$ est indépendamment −$R_4$−Y−$R_7$ ou un radical choisi parmi les radicaux alkyle, alkylèneoxyalkyle, alkylhydroxy, aryle, aralkyle, aralkényle, alcaryle et alkylamidoalkyle ayant de 1 à 30 atomes de

carbone et au moins l'un de $R_7$, $R_2$ et $R_3$ est un radical ayant au moins 8 atomes de carbone et $X^-$ est un anion de chlorure, bromure ou iodure.

**7.** Composé quaternisé de la revendication 6 où m a une valeur de 1 ; R, pris avec −CO et −N−, forme un noyau à 5 ou 6 membres ; $X^-$ est un anion de chlorure et l'un de $R_7$, $R_2$ et $R_3$ est méthyle.

**8.** Composé quaternisé de la revendication 7, où au moins l'un de $R_7$, $R_2$ et $R_3$ est alkyle ayant 8 à 22 atomes de carbone et un autre de $R_7$, $R_2$ et $R_3$ est méthyle.

**9.** Composé quaternisé de la revendication 8, qui est le chlorure de N,N−diméthyl−N−[(2−pyrrolidonyl)méthyl]−N−(3−octadécanamidopropyl)ammonium,
le chlorure de N,N−diméthyl−N−[(2−pyrrolidonyl)méthyl]−N−(3−docosanamidopropyl)ammonium,
le chlorure de N,N−diméthyl−N−[(2−azacycloheptanoyl)−méthyl]−N−(3−octadécanamidopropyl)−ammonium,
le chlorure de N,N−diméthyl−N−[(2−pyrrolidonyl)méthyl]−N−(3−dodécanamidopropyl)ammonium,
le chlorure de N,N−diméthyl−N−[(2−pyrrolidonyl)méthyl]−N−(2−octadécanamidoéthyl)ammonium, ou
le chlorure de N,N−diméthyl−N−[2−pipéridonyl)méthyl]−N−(2−octadécanamidoéthyl)ammonium.

**10.** Composé quaternisé de la revendication 1 ayant pour formule

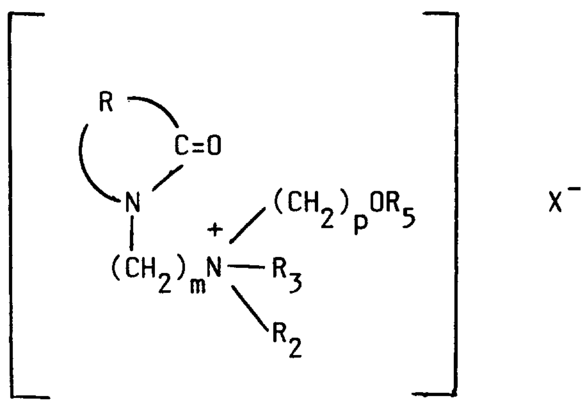

où $X^-$ est un anion de chlorure, bromure ou iodure ; chacun de m et p est un nomhre entier ayant une valeur comprise entre 1 et 4, R est alkylène $C_3$ à $C_9$ où l'alkylène peut être substitué par un alkyle inférieur; $R_5$ est alkyle ou alkényle ayant 1 à 30 atomes de carbone et chacun de $R_2$ et $R_3$ est indépendamment $-(CH_2)_pOR_1$ ou un radical choisi parmi les radicaux alkyle, hydroxyalkyle, aryle, alcaryle, aralkyle, aralkényle et alkylamidoalkyle, ayant 1 à 30 atomes de carbone et au moins l'un de $R_5$, $R_2$ et $R_3$ est un radical contenant 8 à 30 atomes de carbone.

**11.** Composé quaternisé de la revendication 10, où m a une valeur de 1 ; R pris avec −CO et −N− forme un noyau de pyrrolidonyle ou d'azacycloheptanoyle ; $X^-$ est un anion de chlorure et $R_2$ et $R_3$ sont alkyle.

**12.** Composé quaternisé de la revendication 11, où au moins l'un de $R_5$, $R_2$ et $R_3$ est alkyle ayant 8 à 22 atomes de carbone et un autre de $R_5$, $R_2$ et $R_3$ est méthyle.

**13.** Composé quaternisé de la revendication 12 qui est le sel de N,N−diméthyl−N−[(2−pyrrolidonyl)−méthyl]−N−[3−(octadécyloxy)propyl]ammonium,
le sel de N,N−diméthyl−N−[(2−pyrrolidonyl)méthyl]−N−[3−(hexadécyloxy)propyl]ammonium,
le sel de N,N−diméthyl−N−[(2−azacycloheptanoyl)méthyl]−N−[3−(octadécyloxy)propyl]−ammonium,
le sel de N,N−diméthyl−N−[(2−pipéridonyl)méthyl]−N−[3−(dodécyloxy)propyl]ammonium,
le sel de N,N−diéthyl−N−[(2−pyrrolidonyl)méthyl]−N−[3−(octadécyloxy)propyl]ammonium, ou
le sel de N−méthyl−N−[(2−pyrrolidonyl)méthyl]−N,N−bis[3−(octadécyloxy)propyl]ammonium.

**14.** Composé quaternisé de la revendication 1 ayant pour formule

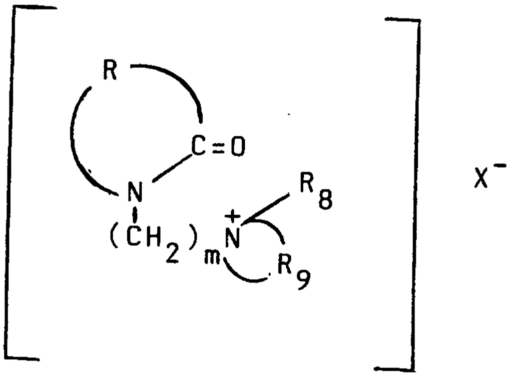

où $X^-$ est un anion de chlorure , bromure ou iodure ; m est un nombre entier ayant une valeur comprise entre 1 et 4 ; R est alkylène ayant 3 à 8 atomes de carbone et est facultativement substitué par un alkyle $C_1$ à $C_4$ ; $R_8$ forme une double liaison dans le noyau hétérocyclique avec l'azote quaternisé ou bien il est choisi dans le groupe consistant en alkyle, hydroxyalkyle, alkylèneoxyalkyle, aryle, alcaryle, aralkyle, aralkényle et alkylamidoalkyle et $R_9$, avec l'atome d'azote quaternisé, forme une structure hétérocyclique de 5 à 14 membres contenant jusqu'à 2 hétéro-atomes choisis dans le groupe consistant en azote, oxygène et soufre.

**15.** Composé de la revendication 14, où R est un alkylène ayant jusqu'à 4 atomes de carbone et est facultativement substitué par alkyle inférieur.

**16.** Composé de la revendication 14, où $R_9$ forme un noyau hétérocyclique saturé avec l'azote quaternisé et où $R_8$ contient 8 à 22 atomes de carbone

**17.** Composé de la revendication 16, où $R_9$ forme un noyau de pyrrolidinyle, un noyau de pipéridinyle ou un noyau de morpholinyle.

**18.** Composé de la revendication 14, où le noyau hétérocyclique formé entre $R_9$ et l'atome d'azote quaternisé contient une double liaison et $R_8$ représente une double liaison dans ledit noyau ou est un groupe lié à l'atome d'azote quaternisé et contient 8 à 22 atomes de carbone.

**19.** Composé de la revendication 18, où $R_9$ forme un noyau d'imidazole et $R_8$ contient 8 à 22 atomes de carbone.

**20.** Composé de la revendication 18, où $R_9$ forme un noyau de pyridinyle ou un noyau d'oxazolidinyle et $R_8$ représente une double liaison dans ledit noyau.

**21.** Composé de la revendication 14, où $R_9$ avec l'atome d'azote quaternisé, forme un noyau hétéromonocyclique de 5 à 10 membres, un noyau hétérobicyclique de 9 à 10 membres ou un noyau hétérotricyclique de 13 à 14 membres.

**22.** Procédé de préparation du composé quaternisé de la revendication 1 qui comprend la réaction d'un lactame ayant pour formule

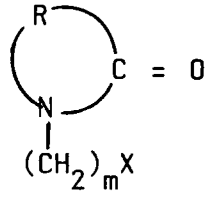

avec un co-réactif d'amine tertiaire ayant pour formule

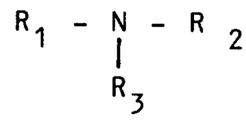

où R, m , $R_1$, $R_2$ et $R_3$ sont tels que définis à la revendication 1 et X est choisi dans le groupe consistant en chloro bromo et iodo ; à une température comprise entre environ 25° et environ 150°C sous une pression de 0 à 0,34 MPa (50 psig).

**23.** Procédé de la revendication 22, où le lactame est la N-chlorométhyl-2-pyrrolidone.

**24.** Procédé de la revendication 23, où l'amine tertiaire a pour formule

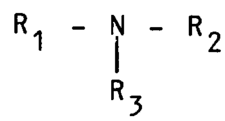

où $R_1$ est méthyle, $R_2$ est méthyle ou un radical contenant 8 à 22 atomes de carbone et $R_3$ est un radical contenant 8 à 22 atomes de carbone.

**25.** Procédé de la revendication 23, où la réaction est effectuée sous la pression atmosphérique à une température comprise entre environ 60° et environ 100°C.

**26.** Procédé de la revendication 22, où l'amine coréactive est choisie dans le groupe de

A. 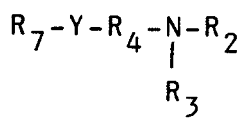 ,

B. 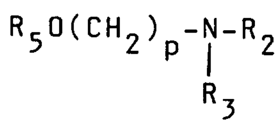 ,

C. 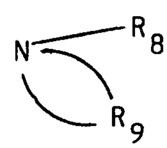 , et

D. 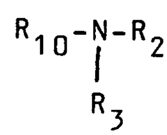

où $R_2$ et $R_3$ sont tels que définis à la revendication 1 ; Y, $R_4$ et $R_7$ sont tels que définis à la revendication 6 ; $R_5$ et p sont tels que définis à la revendication 10 ; $R_8$ et $R_9$ sont tels que définis à la revendication 14 et $R_{10}$ est alkyle ayant 8 à 30 atomes de carbone.

**27.** Utilisation d'un composé selon l'une quelconque des revendications 1 à 21 en tant que conditionneur pour les cheveux dans une formule de shampooing.

**28.** Utilisation selon la revendication 27, où la formule de shampooing contient un sel de N-méthyl-N,N-dioctadécyl-N-[(2-pyrrolidonyl)méthyl]ammonium comme conservateur.

**29.** Utilisation d'un composé selon l'une quelconque des revendications 1 à 21, à raison de 0,5 à 5% en poids d'une formule de shampooing qui contient un agent tensio–actif qui est un lauryl sulfate d'un métal alcalin, un éther de lauryl sulfate d'un métal alcalin, un lauryl sulfate d'ammonium ou un sulfonate d'$\alpha$–oléfine.

**30.** Utilisation d'un composé selon l'une quelconque des revendications 1 à 21, en tant que bactéricide, dans un liquide sujet à une dégradation bactérienne.

**31.** Utilisation du composé de la revendication 14, où le noyau hétérocyclique quaternisé contient un atome de soufre, en tant que bactéricide dans un liquide sujet à une dégradation bactérienne.

**32.** Utilisation d'un composé selon l'une quelconque des revendications 1 à 21, pour augmenter la viscosité d'un liquide.

**33.** Utilisation d'un composé selon l'une quelconque des revendications 1 à 21, pour augmenter la viscosité d'une formule de traitement des cheveux ou de la peau.

**34.** Utilisation d'un composé selon l'une quelconque des revendications 1 à 21, en tant que niveleur de la teinture dans un bain de teinture.

**35.** Utilisation d'un composé selon l'une quelconque des revendications 1 à 21, en tant qu'agent antistatique et d'assouplissement du textile dans une formule de détergent de blanchisserie.

**36.** Utilisation d'un composé selon l'une quelconque des revendications 1 à 21, en tant que conditionneur dans une formule cosmétique.

**37.** Formule cosmétique contenant de 0,05 à 8% en poids d'un composé selon l'une quelconque des revendications 1 à 21.

**38.** Formule de traitement de textiles contenant 0,05 à 8% en poids d'un composé selon l'une quelconque des revendications 1 à 21.